(19) Europäisches Patentamt
European Patent Office
Office européen des brevets

(11) Publication number: **0 186 069 B1**

(12) **EUROPEAN PATENT SPECIFICATION**

(45) Date of publication of patent specification: **19.02.92**

(51) Int. Cl.$^5$: **C12N 15/72**, C12P 21/02, C12N 1/21

(21) Application number: **85115921.0**

(22) Date of filing: **13.12.85**

# (54) New expression control sequence.

(30) Priority: **17.12.84 GB 8431818**

(43) Date of publication of application:
**02.07.86 Bulletin 86/27**

(45) Publication of the grant of the patent:
**19.02.92 Bulletin 92/08**

(84) Designated Contracting States:
**AT BE CH DE FR GB IT LI LU NL SE**

(56) References cited:
**EP-A- 135 094**
**EP-A- 0 067 540**

**CHEMICAL ABSTRACTS, vol. 101, no. 19, 5th November 1984, page 154, abstract no. 164688k, Columbus, Ohio, US; H. BUJARD et al.: "Integration of efficient promoters of the E. coli system into plasmid vectors", & GENE AMPLIF. ANAL. 1983, 3, 65-87**

**NUCLEIC ACIDS RESEARCH, vol. 11, no. 17, 1983, pages 5921-5940, Oxford, GB; J. ROMMENS et al.: "Gene expression: chemical synthesis and molecular cloning of a bacteriophage T5 (T5P25) early promoter"**

**THE EMBO JOURNAL, vol. 3, no. 13, 1984, pages 3143-3148, IRL Press Ltd, Oxford, GB; D. STUEBER et al.: "A novel in vitro transcription-translation system: accurate and efficient synthesis of single proteins from cloned DNA sequences"**

(73) Proprietor: **F. HOFFMANN-LA ROCHE AG**
**Postfach 3255**
**CH-4002 Basel(CH)**

(72) Inventor: **Bujard, Hermann**
**9 Remlerstrasse**
**W-6900 Heidelberg(DE)**
Inventor: **Stüber, Dietrich**
**41 Dürerstrasse**
**W-7888 Rheinfelden(DE)**

(74) Representative: **Lederer, Franz, Dr. et al**
**Lederer, Keller & Riederer Patentanwälte**
**Lucile-Grahn-Strasse 22**
**W-8000 München 80(DE)**

**EP 0 186 069 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new expression control DNA sequences, to expression vectors containing these DNA sequences, to host cells transformed with these expression vectors, to methods for the manufacture of said expression control DNA sequences, expression vectors and transformants, and to methods for producing pro- and eukaryotic proteins by using the new expression control DNA sequences, vectors and transformants.

The level of production of a protein in a host cell is governed by three major factors: the number of copies of its gene within the cell, the efficiency with which those gene copies are transcribed and the efficiency with which the resultant messenger RNA ("mRNA") is translated. Efficiency of transcription and translation is in turn dependent upon the nucleotide sequences which are normally situated ahead of the desired coding sequence or gene. These nucleotide sequences (expression control DNA sequences) define, inter alia, the location at which RNA polymerase interacts (the promoter sequence) to initiate transcription and at which ribosomes bind and interact with the mRNA (the product of transcription) to initiate translation.

Not all such expression control DNA sequences function with equal efficiency. It is thus often of advantage to separate the specific coding sequence or gene for a desired protein from its adjacent nucleotide sequences and to fuse it to other expression control DNA sequences so as to favor higher levels of expression. This having been achieved, the newly-engineered DNA fragment may be inserted into a higher copy number plasmid or a bacteriophage derivative in order to increase the number of gene copies within the cell thereby improving the yield of the desired protein.

Because over-production of even normally non-toxic gene products may be harmful to host cells and lead to decreased stability of particular host-vector systems, an expression control DNA sequence, in addition to improving the efficiency of transcription and translation of cloned genes, should be made controllable so as to allow modulation of expression during bacterial growth. For example, controllable expression control DNA sequences are ones that may be switched off to enable the host cells to propagate without excessive build-up of gene products and later be switched on to promote the expression of large amounts of the desired protein products, which are under the control of those expression control DNA sequences.

Several expression control DNA sequences, which satisfy some of the criteria set forth above, have been employed to express DNA sequences and genes coding for proteins and polypeptides in bacterial hosts. These include, for example, the operator, promoter and ribosome binding and interaction sequences of the lactose operon of E.coli (e.g., K. Itakura et al., "Expression in Escherichia coli of a chemically synthesized gene for the hormone somatostatin", Science, 198, pp. 1056-1063 [1977]; D.V. Goeddel et al., "Expression in Escherichia coli of chemically synthesized genes for human insulin", PNAS USA, 76, pp. 106-110 [1979]), the corresponding sequences of the tryptophan synthetase system of E. coli (J.S. Emtage et al., "Influenza antigenic determinants are expressed from Haemagglutinin genes cloned in Escherichia coli", Nature, 283, pp. 171-174 [1980]; J.A. Martial et al., "Human growth hormone: Complementary DNA cloning and expression in bacteria", Science, 205, pp. 602-607 [1979]) and the major operator and promoter regions of phage λ (H. Bernard et al., "Construction of plasmid cloning vehicles that promote gene expression from the bacteriophage lambda $P_L$ promoter", Gene, 5, pp. 59-76 [1979]; European patent application, publication no. 41767).

The present invention provides a novel and improved expression control DNA sequence comprising a coliphage T5 promoter combined with a DNA sequence which permits the control of promoter activity (regulating sequence).

More precisely the present invention allows to combine a DNA sequence which permits the control of promoter activity with a coliphage T5 promoter while maintaining at the same time the highly efficient promoter function of the coliphage T5 promoter.

In this invention T5 promoters are defined as promoter function mediating DNA sequences occurring in genomes of the coliphage T5 family and functional combinations derived from such sequences.

Naturally occurring T5 promoters are known to be efficient transcriptional initiation signals (A. von Gabain and H. Bujard, "Interaction of E.coli RNA polymerase with promoters of coliphage T5", Molec. gen. Genet., 157, pp. 301-311 [1977]) with the following main properties:

(i) they exhibit a high forward rate constant in the complex formation between the promoter sequence and E.coli RNA polymerase (A. von Gabain and H. Bujard, "Interaction of Escherichia coli RNA polymerase with promoters of several coliphage and plasmid DNAs", PNAS, 76, pp. 189-193 [1979]);

(ii) they initiate RNA synthesis in vivo and in vitro with unusually high rates;

(iii) they contain conserved sequences in 5 regions of the RNA polymerase binding site (H. Bujard et al., "Integration of efficient promoters of the E.coli system into plasmid vectors", in Gene Amplification and Analysis, Vol. 3: Expression of cloned genes in prokaryotic and eukaryotic cells, eds. T.S. Papas, M. Rosenberg, and J.G. Chirikjian; Elsevier New York-Amsterdam-Oxford, pp. 65-87 [1983]) and

(iv) cloned in expression vehicles these promoters are not regulatable.

T5 promoters useful in the present invention are those of the "preearly" "early" and "late" expression class of the phage, especially the sequences described in the disseration of R. Gentz, Universität Heidelberg, 1984: $P_{207}$, $P_{25}$ (named $P_{N25}$ in the present specification), $P_{26}$, $G_{30}$, $P_{28a}$ (named $P_{28}$ in Bujard et al. supra), $P_{28b}$, $G_{22}$, $G_5$, $G_{25}$, $G_{28}$, $G_{20}$.

The DNA sequence of some of the preferred T5 promoters mentioned above are indicated in Table I below:

## Table I

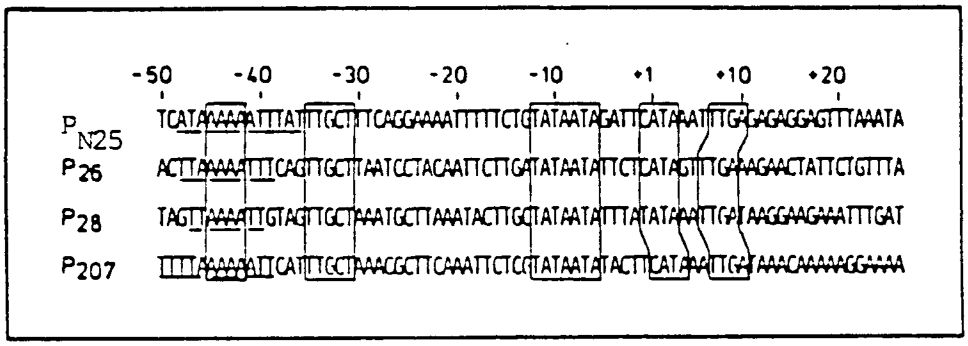


Table 1 shows the nucleotide sequence of these four T5 promoters reacting rapidly with E.coli RNA polymerase. Regions of major homology are boxed. Most striking are the homologies in -10, -33, -43, and +7 regions, as well as the precise spacing (18 bp) between -10 and -33 homologies. The underlined sequences centering around -43 indicate regions where a strong selection against GC base pairs occurs.

DNA sequences which permit the control of the promoter activity are known in the art. They occur in operons (regulatable expression unit) of various types (J.H. Miller and W.S. Reznikoff, "The operon", Cold Spring Harbor Laboratory [1980]). One such type is a negatively controlled expression unit whose essential element is an operator sequence which interacts with a repressor protein. The coding sequence for the repressor protein can be located on a plasmid carrying the operator, or on a separate vector or in the host chromosome.

Preferred DNA sequences which permit the control of the promoter activity, used in this invention, are operator/repressor systems. Especially preferred is the system of the lac operon consisting of the nature lac operator or functional sequences derived therefrom and repressor encoding DNA sequences producing natural or modified repressor molecules (J.H. Miller and W.S. Reznikoff, supra).

The expression control DNA sequences of the present invention can be obtained as follows:

A coliphage T5 promoter sequence can be combined in a manner well known in the art with one or more sequence(s) which permit the control of promoter activity (regulating sequence) to a functional unit whereby the position of the regulating sequences e.g., an operator can vary. In such combinations the regulating sequence can be positioned within or outside the T5 promoter sequence. Thus, a regulating sequence may be integrated within the promoter, may be partially overlapping with or may preceed or follow the promoter at various distances without any overlap. Preferably the regulating sequence overlaps the promoter sequence between position +1 and +20, -13 and -30 and/or -34 and -50 (nomenclature as in table I). Further preferred positions are regions "downstream" of position +20 up to +200. Especially preferred sites are those where the regulating sequence e.g., the lac operator sequence, overlaps the T5 promoter sequence until position +2 in analogy to the lac operon, in E.coli, as shown in Fig. 7.

The expression control DNA sequence of the present invention can be obtained in accordance with methods well-known in DNA chemistry including total chemical synthesis of the respective DNA sequence.

In the preferred embodiment of this invention the lac operator can be negatively regulated by the lac repressor protein which is coded by the lac I-gene. To obtain proper amounts of repressor molecules inside the cell the corresponding gene can be overexpressed by conventional methods, for example by integrating the lac $I^q$ gene (J.H. Miller and W.S. Reznikoff, supra; M.P. Calos, "DNA sequence of a low-level promoter of the lac repressor gene and an 'up' promoter mutation, Nature, 274, pp. 762-765 [1978]) into a vector comprising the above expression control sequence.

The expression sequence of this invention can be introduced in any convenient expression vector of plasmid or phage origin in a manner known per se. Convenient expression vectors of plasmid or phage origin are mentioned e.g., in the laboratory manual "Molecular Cloning" by Maniatis et al., Cold Spring Harbor Laboratory, 1982.

Members of the pDS1-family of plasmids were shown to be suitable for the stable integration of transcriptional signals of exceptional strength (D. Stüber and H. Bujard, "Transcription from efficient promoters can interfere with plasmid replication and diminish expression of plasmid specified genes", The EMBO Journal, 1, pp. 1399-1404 [1982]).

Therefore the preferred vectors used in this invention are those of the pDS1 family. These plasmids are derived from plasmid pBR322 and comprise e.g., pDS1,$t_o1^+$ and pDS1, $t_o2^+$ resulting in plasmid constructions like pDS2/$P_{N25}x_{/0}$,$t_o2^+$ and pDS3/$P_{N25}x_{/0}$,$t_o2^+$.

E.coli strains containing plasmids useful for such constructions (E.coli M15 transformed with pDS1,$t_o1^+$; pDS1,$t_o2^+$; pDSIX,1) were deposited at Deutsche Sammlung von Mikroorganismen (DSM) in Göttingen on December 11, 1984, the accession nos. being DSM 3135, DSM 3136, DSM 3137 respectively.

The DNA sequences that may be expressed by the expression vectors of this invention may be selected from a large variety of DNA sequences that encode prokaryotic or eukaryotic polypeptides in vivo or in vitro. For example, such sequences may encode enzymes, hormones, polypeptides with immunomodulatory, anti-viral or anti-cancer properties, antibodies, antigens, vaccines and other useful polypeptides of prokaryotic or eukaryotic origin.

Examples of proteins which can be expressed by using the improved expression control system of the present invention are dihydrofolate reductase, chloramphenicol acetyltransferase, malaria surface antigens, lymphokines like IL-2, interferons $\alpha$, $\beta$ and $\gamma$, insulin and insulin precursors, growth hormones, tissue plasminogen activator or human renin.

Methods for expressing DNA sequences coding for prokaryotic or eukaryotic proteins using the expression vectors of this invention are well known (Maniatis et al., supra). They include transforming an appropriate host with the expression vector having the desired DNA sequence operatively linked to the expression control sequence of the vector, culturing the host under appropriate conditions of growth and isolating the desired polypeptide from the culture. Those of skill in the art may select from these known methods those that are most effective for a particular gene expression without departing from the scope of this invention.

The selection of a particular host for use in this invention is dependent upon a number of factors recognized by the art. These include, for example, compatibility with the chosen expression vector, toxicity of the proteins encoded for by the hybrid plasmid, ease of recovery of the desired protein, expression characteristics, biosafety and costs. Within these general guidelines, examples of useful bacterial hosts are gram-negative and gram-positive bacteria, especially strains of E.coli and B.subtilis. The most preferred host cell of this invention is E.coli M15 (described as DZ291 in M.R. Villarejo et al., "$\beta$-Galactosidase from termination and deletion mutant strains", J. Bacteriol., 120, pp. 466-474 [1974]). However other E.coli strains such as E.coli 294 (ATCC No. 31446) and E.coli RR1 (ATCC No. 31343) can also be used.

The present invention will be better understood on the basis of the following examples when considered in connection with the following figures:

The abbreviations and symbols used are: B, E, H, P, X and Xb which indicate sites for restriction endonucleases BamI, EcoRI, HindIII, PstI, XhoI and XbaI, respectively. represents promoters; represents ribosomal binding sites; represents terminator $t_o$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), $\beta$-lactamase (bla) and lac repressor (lacI) or for parts of the coding region for chloramphenicol acetyltransferase (cat'); represents parts of the coding region for $\beta$-galactosidase (lac Z').

Figure 1. Part a) is a schematic drawing of the plasmid pDS1,$t_o1^+$. The nucleotide sequence of the EcoRI/XbaI-fragment containing the dhfr-gene, terminator $t_o$ and the cat-gene is displayed in part b). Here the restriction endonuclease sites indicated in part a) are overlined. In addition the pBR322 entity of pDS1,$t_o1^+$ is schematically shown, where the given numbers refer to the nucleotide sequence of pBR322 (J.G. Sutcliffe, "Complete nucleotide sequence of the Escherichia coli plasmid pBR322", Cold Spring Harbor Symp. Quant. Biol., 43, pp. 77-90 [1979].

Figure 2 is a schematic drawing of the plasmid pDS1/$P_{N25}$,$t_o1^+$. The nucleotide sequence of the XhoI-fragment carrying promoter $P_{N25}$ is displayed. The site of initiation and the direction of transcription are indicated by the arrow.

Figure 3 is a schematic drawing of the plasmid pEX07 lac op 2. The nucleotide sequence of the EcoRI/HindIII-fragment carrying the lac operator is displayed.

Figure 4. Part a) is a schematic drawing of the plasmid pDS1,$t_o2^+$. The nucleotide sequence of the XhoI/XbaI-fragment containing the dhfr-gene, the cat-gene and terminator $t_o$ is displayed in part b). Here the restriction endonuclease sites indicated in part a) are overlined. In addition the pBR322 entity of pDS1,$t_o2^+$ is schematically shown, where the given numbers refer to the nucleotide sequence of pBR322 (J.G. Sutcliffe, supra).

Figure 5. Part a) is a schematic drawing of the plasmid pDSIX,1. The nucleotide sequence of the EcoRI/XbaI-fragment containing the lac I-gene, parts of the lac Z-gene (lac Z') and the cat-gene is displayed in parts b) and c). Here the restriction endonuclease sites indicated in part a) are overlined. In addition the pBR322 entity of pDSIX,1 is schematically shown, where the given numbers refer to the nucleotide sequence of pBR322 (J.G. Sutcliffe, supra).

Figure 6a,b,c is a schematic outline of the construction of the promoter/operator fusion $P_{N25}x_{/0}$ embedded in the plasmid pDS2/$P_{N25}x_{/0}$,$t_o2^+$.

Figure 7 displayes the nucleotide sequence of the XhoI/EcoRI fragment containing the promoter/operator fusion $P_{N25}x_{/0}$. The site of initiation and the direction of transcription are indicated by the arrow.

Figure 8a,b,c is a schematic outline of the construction of plasmids pDSXII,3, pDS3/$P_{N25}x_{/0}$,$t_o2^+$ and pDSXIII,1.

Figure 9 is an electropherogram monitoring protein synthesis in the absence or presence of IPTG in E.coli M15 harboring plasmids pDSXII,3 (lane 1), pDS2/$P_{N25}x_{/0}$,$t_o2^+$ (lanes 2 and 3) or pDS3/$P_{N25}x_{/0}$,$t_o2^+$ (lanes 4-7).

The construction of the promotor/operator fusion $PN_{N25}x_{/0}$ of this invention is described more in detail in the following examples:

Example 1

Description of plasmids used for the construction of promoter $P_{N25}x_{/0}$

A. Principles

pDS1, $t_o1^+$ (figure 1) and pDS1, $t_o2^+$ (figure 4) were chosen as vectors not only for the integration of promoter $P_{N25}$ and the construction of the fusion between promoter $P_{N25}$ and the lac operator but also for the demonstration of the function of the resulting promoter/operator fusion $P_{N25}x_{/0}$.

Although there are several well defined sources for promoter $P_{N25}$, plasmid pDS1/$P_{N25}$,$t_o1^+$ (figure 2) carrying this element on an EcoRI-fragment was chosen as source for promoter $P_{N25}$ whereas pEX07 lac op 2 (figure 3; H. Weiher, "Untersuchungen zur Struktur und Funktion von E.coli Promotoren: Variation des Lac Promoters durch gezielte Neukonstruktion von Teilsequenzen und Mutagenese", Ph.D. thesis, University of Heidelberg, FRG [1980]) comprising 21 basepairs of the lac operator was chosen to provide the operator entity in the promoter/operator fusion. The lac repressor is encoded by the lac I gene. Since a promoter is efficiently repressed by binding of repressor to the operator only if sufficient amounts of repressor molecules are present, the lac $I^q$ allele was used with a mutant promoter resulting in an increased transcription of the gene to provide enough repressor molecules. As source of this mutant lac I gene plasmid pIX,1 was chosen (figure 5).

B. Plasmid pDS1,$t_o1^+$

The part of pDS1,$t_o1^+$ (figure 1; D. Stüber and H. Bujard, supra) between the sites for restriction endonucleases XbaI and EcoRI containing the region required for DNA replication and maintenance of the plasmid in the cell and the entire gene for $\beta$-lactamase conferring resistance to ampicillin is pBR322 derived (F. Bolivar et al., "Construction and characterization of new cloning vehicles II. A multi-purpose cloning system", Gene, 2, pp. 95-113 [1977]; J.G. Sutcliffe, supra). The remaining part of the plasmid carries sites for restriction endonucleases XhoI, EcoRI and BamHI followed by the coding region for dihydrofolate reductase of mouse AT-3000 cell line (A.C.Y. Chang et al., "Phenotypic expression in E. coli of a DNA sequence coding for mouse dihydrofolate reductase", Nature, 275, pp. 617-624 [1978]; J.N. Masters and G. Attardi, "The nucleotide sequence of the cDNA coding for the human dihydrofolic acid reductase", Gene, 21, pp. 59-63 [1983]), by the terminator $t_o$ of phage lambda (E. Schwarz et al., "Nucleotide sequence of cro, cII and part of the O gene in phage $\lambda$ DNA", Nature, 272, pp. 410-414 [1978]; D. Stüber and H. Bujard, supra) and by the promoter-free gene for chloramphenicol acetyltransferase (R. Marcoli et al., "The DNA sequence of an IS1-flanked transposon coding for resistance to chloramphenicol and fusidic acid", FEBS Letters, 110, pp. 11-14 [1980]).

C. Plasmid pDS1/$P_{N25}$,$t_o1^+$

Plasmid pDS1/$P_{N25}$,$t_o1^+$ (figure 2) corresponds to pDS1,$t_o1^+$ (figure 1) but contains on an XhoI-fragment promoter $P_{N25}$ of E.coli phage T5 (A. von Gabain and H. Bujard, supra; D. Stüber et al., "Electron microscopic analysis of in vitro transcriptional complexes: Mapping of promoters of the coliphage T5 genome", Molec. gen. Gen., 166, pp. 141-149 [1978]; H. Bujard et al., supra).

D. Plasmid pEX07 lac op 2 Plasmid pEX07 lac op 2 (figure 3; H. Weiher, supra) is a derivative of pBR322 (F. Bolivar et al., supra; J.G. Sutcliffe, supra) where the EcoRI/HindIII-fragment of pBR322 is replaced by an EcoRI/HindIII-fragment containing 21 basepairs of the lac operator sequence (J.H. Miller and W.S. Reznikoff, supra).

E. Plasmid pDS1,$t_o2^+$

The part of pDS1,$t_o2^+$ (figure 4; D. Stüber and H. Bujard, supra) between the sites for restriction endonucleases XbaI and XhoI containing the region required for DNA replication and maintenance of the plasmid in the cell and the entire gene for β-lactamase confering resistance to ampicillin is pBR322 derived (F. Bolivar et al., supra; J.G. Sutcliffe, supra). The remaining part of the plasmid carries sites for restriction endonucleases EcoRI and BamHI followed by the coding region for dihydrofolate reductase of mouse AT-3000 cell line (A.C.Y. Chang et al., supra; J.N. Masters and G. Attardi, supra), by the promoter-free gene for chloramphenicol acetyltransferase (R. Marcoli et al., supra) and by the terminator $t_o$ of phage lambda (E. Schwarz et al., supra).

F. Plasmid pDSIX,1

Plasmid pDSIX,1 (figure 5) carries the identical part of pBR322 as plasmid pDS1,$t_o1^+$ (figure 1). In addition it contains the entire gene for the lac repressor (P.J. Farabaugh, "Sequence of the lacI gene", Nature, 274, pp. 765-769 [1978]) with the promoter mutation $I^q$ (M.P. Calos, supra) followed by sites for restriction endonucleases EcoRI and XhoI, by the regulatory and part of the coding region of the gene for β-galactosidase (A. Kalnins et al., "Sequence of the lac Z gene of Escherichia coli", The EMBO J., 2, pp. 593-597 [1983]; R. Gentz et al., "Cloning and analysis of strong promoters is made possible by the downstream placement of a RNA termination signal", PNAS, 78, pp. 4936-4940 [1981]) and by the promoter-free gene for chloramphenicol acetyltransferase (R. Marcoli et al., supra).

Example 2

Construction of the plasmid pDs2/$P_{N25}x_{/0}$,$t_o2^+$ carrying the promoter/operator fusion $P_{N25}x_{/0}$

The promoter/operator fusion $P_{N25}x_{/0}$ was prepared and embedded in pDS1,$t_o2^+$ to give pDS2/$P_{N25}x_{/0}$,$t_o2^+$ in a sequence of steps. These are depicted in figure 6 and more fully described below.

A. Preparation of the promoter entity of the promoter/operator fusion

a) Isolation of the EcoRI-fragment containing $P_{N25}$

To isolate the EcoRI-fragment carrying promoter $P_{N25}$, 32.5 μg of plasmid pDS1/$P_{N25}$,$t_o1^+$ were digested with EcoRI (figure 6a) and after extraction with phenol followed by treatment with ether and subsequent precipitation with ethanol the DNA fragments were separated by electrophoresis in a 6% polyacrylamide gel. Following visualization of the DNA by staining with ethidium bromide, the fragment carrying $P_{N25}$ was cut out of the gel and eluted by shaking the mashed gel slice for 10 hours in a buffer containing 2.5 mM Tris/HCl (pH 7.6) and 0.25 mM EDTA. After removal of the gel pieces by centrifugation, the resulting supernatant was extracted with phenol, treated with ether and the DNA fragment collected by precipitation with ethanol. 1.3 μg of the promoter-fragment were isolated in this way.

b) Limited digestion of the EcoRI-fragment with HinfI

1 μg of the EcoRI-fragment was incubated in a volume of 120 μl in a buffer containing 100 mM sodium chloride with 21 units HinfI for 9 minutes at 37°C. The enzyme was inactivated by incubation of the mixture for 7 minutes at 65°C and withdrawn by three extractions with phenol. After removal of the residual phenol by extraction with ether the DNA was precipitated with ethanol and stored in a buffer containing 5 mM Tris/HCl (pH 7.6), 0.5 mM EDTA and 100 mM sodium chloride. Analysis of the DNA by electrophoresis in a 6% polyacrylamid gel revealed that about 60% of the EcoRI-fragment molecules had been cleaved by HinfI.

c) Extending the recessed 3'-ends with DNA polymerase I

0.17 μg of the HinfI digested DNA was incubated in a volume of 25 μl in a buffer consisting of 100 mM potassium phosphate (pH 7.0), 100 mM potassium chloride, 7 mM magnesium chloride, 25 μM each of the four deoxynucleoside-triphosphates with 5 units of DNA polymerase I for 40 minutes at 20°C. After removal of the enzyme by extraction with phenol, the solution was chromatographed through Sephadex® G75 equilibrated in a buffer of pH 7.7 consisting of 2.5 mM Tris, 0.25 mM EDTA and 50 mM potassium acetate. DNA containing fractions were combined before the DNA was precipitated with ethanol and stored in ligation buffer supplemented with 50 mM sodium chloride. The resulting solution with 0.08 µg DNA contained fragment 1 (figure 6a).

B. Preparation of the operator entity of the promoter/operator fusion

a) Limited digestion of pEX07 lac op 2 14.5 µg of plasmid pEX07 lac op 2 (figure 6b) were incubated in a volume of 250 µl with 1.5 units EcoRI for 50 minutes at 37°C. The enzyme was inactivated by incubation for 7 minutes at 65°C and withdrawn by extraction with phenol. After removal of residual phenol with ether the DNA was precipitated with ethanol and stored in a buffer consisting of 2.5 mM Tris/HCl (pH 7.6) and 0.25 mM EDTA. Analysis of the DNA by electrophoresis in a 6% polyacrylamide gel revealed that about 50% of the plasmid molecules had been linearized.

b) Removal of the 5'-protruding ends with S1 nuclease To remove the extended 5'-ends, the EcoRI digested DNA was treated with the single-strand specific nuclease S1 (figure 6b). In three separate experiments 1.7 µg DNA were incubated in a volume of 50 µl in a buffer containing 280 mM sodium chloride, 30 mM sodium acetate (pH 4.4), 4.5 mM zinc acetate and 20 µg /ml single-stranded DNA of phage fd with 160 units, 800 units and 4000 units S1 nuclease (Boehringer, Mannheim, FRG) for 30 minutes at 20°C. The reactions were stopped by adding ammonium acetate and EDTA to final concentrations of 0.5 M and 10 mM, respectively, and incubation for 7 minutes at 65°C. After removal of the protein by extraction with phenol followed by treatment with ether, the three mixtures were combined and the resulting solution chromatographed through Sephadex® G75 equilibrated in a buffer of pH 7.0 consisting of 2.5 mM Tris/HCl, 0.25 mM EDTA and 50 mM potassium acetate. DNA containing fractions were combined before the DNA was precipitated with ethanol and stored in ligation buffer. The resulting solution with 4.3 µg DNA contained fragment 2 (figure 6b).

C. Preparation of plasmid pDS1,$t_o2^+$ accepting the promoter/operator fusion

pDS1,$t_o2^+$ (figure 4) was chosen as vector to accept the promoter/operator fusion. Therefore, 5.1 µg DNA of this plasmid were digested to completion with restriction endonucleases HindIII and XhoI (figure 6c). After inactivation of the enzymes by incubation for 7 minutes at 65°C and removal of the protein by extraction with phenol followed by treatment with ether, the DNA was precipitated with ethanol and stored in a buffer consisting of 2.5 mM Tris/HCl (pH 7.6) and 0.25 mM EDTA.

D. Construction of pDS2/$P_{N25}x_{/0}$,$t_o2^+$

To construct pDS2/$P_{N25}x_{/0}$,$t_o2^+$ the promoter entity (fragment 1) and the operator entity (fragment 2) of $P_{N25}x_{/0}$ were ligated together and the resulting fusion subsequently embedded in pDS1,$t_o2^+$ (figure 6c). For the fusion of promoter and operator 0.05 µg of the DNA mixture containing fragment 1 and 3.5 µg of the DNA mixture containing fragment 2 were incubated in ligation buffer supplemented with 25 mM sodium chloride with 5 units T4-DNA ligase for 7 hours at 15°C. After inactivation of the enzyme by incubation for 7 minutes at 65°C, the ligation mixture was incubated in a volume of 100 µl with 20 units HindIII and 15 units XhoI for 1 hour at 37°C to obtain the promoter/operator fusion as an XhoI/HindIII fragment. After inactivation of the restriction endonucleases at 65°C and their removal by extraction with phenol followed by treatment with ether, the DNA was precipitated with ethanol and redissolved in ligation buffer supplemented with 25 mM potassium chloride. 0.25 µg plasmid pDS1,$t_o2^+$ digested with HindIII as well as XhoI and 1.3 units T4-DNA ligase were added and the mixture incubated for 4 hours at 15°C before the enzyme was inactivated by incubation for 7 minutes at 65°C.

E.coli C600 ($CaCl_2$ competent) was transformed with half of the above prepared ligation mixture under appropriate conditions. Transformants were selected at 37°C on Minimal Agar Plates based on M9-medium (J.H. Miller, "Experiments In Molecular Genetics", Cold Spring Harbor Laboratory, pp. 431-432 [1972]) supplemented with 0.2% glucose, 0.5% casein hydrolysate, 10 mg/liter vitamin B1, 40 mg/liter X-gal (5-bromo-4-chloro-3--indolyl-β-D-galactoside) and 10-200 µg/ml chloramphenicol.

Only transformants containing plasmid pDS1,$t_02^+$ with a promoter integrated in front of the promoter-free gene for chloramphenicol acetyltransferase were expected to grow on plates with concentrations of chloramphenicol of more than 10 µg/ml. Furthermore, transformants containing pDS1,$t_02^+$ with $P_{N25}x_{/0}$ integrated in front of this indicator gene were expected to become blue on these plates, due to the induction of the host lac system by binding of the lac repressor molecules to the operator sequence present in the plasmids and subsequent cleavage of the colourless X-gal to a blue derivative. Therefore, 18 blue transformants from plates with 50 µg/ml or higher concentrations of chloramphenicol were selected and cultures grown at 37°C in LB-medium containing 100 µg/ml ampicillin. DNA from these cultures was isolated using standard procedures and analyzed for its size and the presence of sites for restriction endonucleases EcoRI, HindIII, HinfI and XhoI. One plasmid displaying the expected patterns after electrophoresis of restriction fragments in 6% polyacrylamid gels was designated pDS2/$P_{N25}x_{/0}$,$t_0$$2^+$ (figure 6c). Sequence analysis of the HindIII-XhoI fragment of this plasmid confirmed the expected sequence for promoter $P_{N25}x_{/0}$ as depicted in figure 7.

## Example 3

### Construction of plasmids pDSXII,3, pDS3,$P_{N25}x_{/0}$,$t_0$$2^+$ and pDSXIII,1 containing promoter $P_{N25}x_{/0}$

In addition to pDS2/$P_{N25}x_{/0}$,$t_0$$2^+$ (lacking repressor gene) three more plasmids containing the promoter/operator fusion $P_{N25}x_{/0}$, namely pDSXII,3 (intermediate for the manufacture of pDS XIII,1), pDS3/$P_{N25}x_{/0}$,$t_0$$2^+$ (as an example of plasmids according to the invention) and pDSXIII,1 (with a partly deleted cat-gene lacking RBS; as a negative control for comparative purposes), were designed to demonstrate the function of this element. The construction of these plasmids is depicted in figure 8 and more fully described below.

A. Construction of plasmid pDSXII,3 Plasmid pDSXII,3 was derived from pDS2/$P_{N25}x_{/0}$,$t_0$$2^+$ by eliminating the EcoRI-fragment of this plasmid containing the single site for restriction endonucleases HindIII and part of the gene for chloramphenicol acetyltransferase (figure 8a). For that purpose 0.45 $\mu$g of pDS2/$P_{N25}x_{/0}$,$t_0$$2^+$ were digested to completion with restriction endonuclease EcoRI. After removal of the enzyme by extraction with phenol, the solution was chromatographed through Sephadex G75 equilibrated in a buffer of pH 7.6 consisting of 2.5 mM Tris/HCl and 0.25 mM EDTA. DNA containing fractions were combined and the resulting solution was concentrated by lyophilization. 0.13 $\mu$g of the plasmid digested with EcoRI were incubated in a volume of 100 $\mu$l in ligation buffer with 1.3 units T4-DNA ligase for 5 hours at 15 °C before the enzyme was inactivated by incubation for 7 minutes at 65 °C.

E.coli M15 ($CaCl_2$ competent) was transformed with 0.07 $\mu$g of the ligated DNA under appropriate conditions. Transformants were selected at 37 °C on Minimal-Agar plates based on M9-medium (J.H. Miller, supra) supplemented with 0.2% glucose, 0.5% casein hydrolysate, 10 mg/liter vitamin B1, 40 mg/liter X-gal and 1 mM IPTG (Isopropylthiogalactoside) and containing either 100 $\mu$g/ml ampicillin or 50 $\mu$g/ml chloramphenicol.

Since loss of the EcoRI-fragment comprising part of the gene for chloramphenicol acetyltransferase results in loss of resistance to chloramphenicol, plates with ampicillin were found to have about 40 times more transformants than plates with ampicillin. 4 transformants resistant to ampicillin were selected and cultures grown in LB-medium containg 100 $\mu$g/ml ampicillin. DNA from these cultures was isolated using standard procedures and analyzed for its size and presence of sites for restriction endonucleases EcoRI, HindIII, HinfI, PstI and XhoI. One plasmid displaying the expected patterns after electrophoresis in 6% polyacrylamid-gels was designated pDSXII,3 (figure 8a).

B. Construction of plasmids pDS3/$P_{N25}x_{/0}$,$t_o$$2^+$ and pDSXIII,1

Plasmids pDS3/$P_{N25}x_{/0}$,$t_o$$2^+$ and pDSXIII,1 were derived by combining appropriate fragments of plasmids pDSIX,1 and pDS2/$P_{N25}x_{/0}$,$t_o$$2^+$ (figure 8b) or pDSXII,3 (figure 8c), respectively. For that purpose, 1.25 $\mu$g of these three plasmids were digested in three separate reactions to completion with restriction endonucleases PstI and XhoI. After removal of these enzymes by extraction with phenol, the three solutions were chromatographed through Sephadex G75 equilibrated in a buffer of pH 7.6 consisting of 2.5 mM Tris/HCl and 0.25 mM EDTA. DNA containing fragments of each experiment were combined and the resulting solutions concentrated by lyophilization.

In two separate reactions 0.13 $\mu$g digested DNA of each of the plasmids pDSIX,1 and pDS2/$P_{N25}x_{/0}$,$t_o$$2^+$ or pDSIX,1 and pDSXII,3 were incubated in a volume of 60 $\mu$l in ligation buffer with 1.3 units T4-DNA ligase for 10 hours at 15 °C before the enzyme was inactivated by incubation for 7 minutes at 65 °C. Again in two separate reactions E.coli M15 ($CaCl_2$ competent) was transformed with 0.12 $\mu$g of the ligated DNA under appropriate conditions. Transformants were selected at 37 °C on Minimal-Agar plates based on M9-medium (J.H. Miller, supra) supplemented with 0.2% glucose, 0.5% casein hydrolysate, 10 mg/liter vitamin B1, 40 mg/liter X-gal and 1 mM IPTG and containing 100 $\mu$g/ml ampicillin. Six white transformants of each transformation were selected and cultures grown in LB-medium containing 100 $\mu$g/ml ampicillin. DNA of these cultures was isolated using standard procedures and analyzed for its size and presence of sites for restriction endonucleases EcoRI, HindIII, HinfI, PstI and XhoI. Two plasmids derived from pDS2/$P_{N25}x_{/0}$,$t_o$$2^+$ and pDSXII,3 displaying the expected patterns after electrophoresis in 6% polyacrylamid gels were designated pDS3/$P_{N25}x_{/0}$,$t_o$$2^+$ (figure 8b) and pDSXIII,1 (figure 8c), respectively.

Example 4

Expression of chloramphenicol acetyltransferase in plamids containing promoter/operator fusion $P_{N25}x_{/0}$

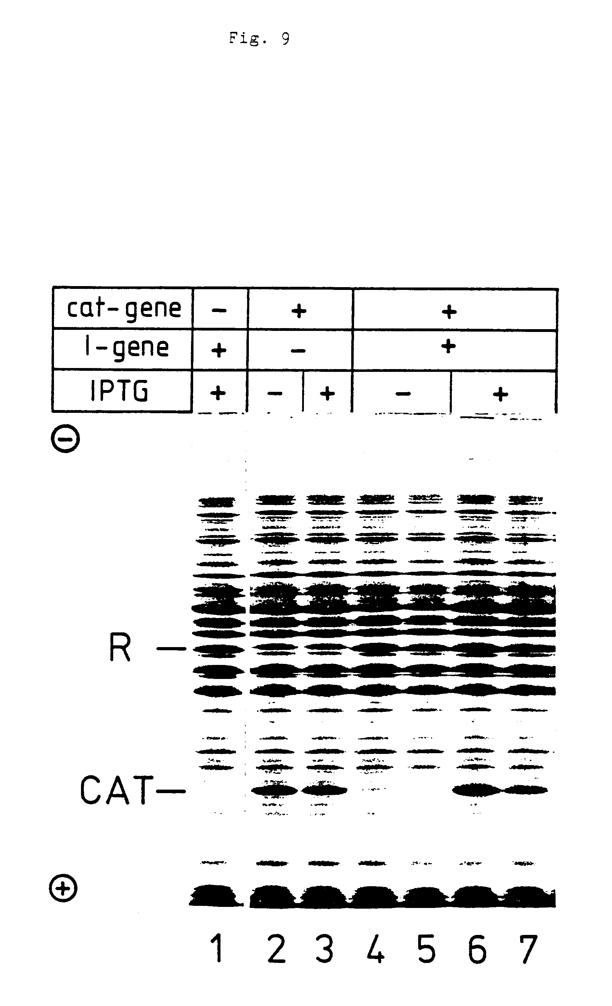

To demonstrate the usefulness of promoter/operator fusion $P_{N25}x_{/0}$, the synthesis of chloramphenicol acetyltransferase in cells harboring plasmids containing $P_{N25}x_{/0}$ was monitored. E.coli M15 cells transformed with either pDS2/$P_{N25}x_{/0}$,$t_o2^+$, pDSXIII,1 or pDS3/$P_{N25}x_{/0}$, $t_o2^+$ were grown in the presence or absence of 1 mM IPTG in LB-medium containing 100 $\mu$g/ml ampicillin at 37° C to a density of $1.7 \cdot 10^9$ cells/ml. $1.5 \cdot 10^8$ cells were collected by centrifugation and resuspended in sample buffer containing 1% SDS, 1% $\beta$-mercaptoethanol, 10% glycerol and 62.5 mM Tris/HCl (pH 6.8). Samples were boiled for 5 minutes, chilled on ice, centrifuged at 12 000 xg for 30 seconds and electrophoresed in a SDS-containing polyacrylamide gel (12.5% acrylamide) according to the procedure of U. Laemmli, "Cleavage of structural proteins during the assembly of the head of Bacteriophage T4", Nature, 227, pp. 680-682 [1970]. After staining of the proteins with Coomassie brillant Blue R-250 the unbound dye was removed from the gel. A fotograph of this gel is shown in figure 9.

Comparing now lanes 1, 2 and 3 of figure 9 it becomes obvious that as expected pDSXIII,1 (lane 1) encodes lac repressor (R) but not chloramphenicol acetyltransferase (CAT) whereas in cells transformed with pDS2/$P_{N25}x_{/0}$,$t_o2^+$ (lanes 2 and 3) the repressor is absent and chloramphenicol acetyltransferase is produced in large amounts independent of the presence of IPTG in the medium. Although pDS3/$P_{N25}x_{/0}$,$t_o2^+$ differs from pDS2/$P_{N25}x_{/0}$,$t_o2^+$ only in comprising the gene for the lac repressor (figure 8b), in cells transformed with pDS3/$P_{N25}x_{/0}$,$t_o2^+$ only unvisible amounts of chloramphenicol acetyltransferase are produced in the absence of IPTG (compare lanes 4 and 5 with lanes 1, 2 and 3). However, in cells harboring pDS3/$P_{N25}x_{/0}$,$t_o2^+$ and grown in the presence of IPTG essentially the same amount of chloramphenicol acetyltransferase is produced as in cells containing pDS2/$P_{N25}x_{/0}$,$t_o2^+$ (compare lanes 6 and 7 with lanes 2 and 3).

The results described above show, that promoter $P_{N25}x_{/0}$ is repressed in the presence of lac repressor and is induced essentially to full activity by addition of the inducer IPTG.

## Claims

**Claims for the following Contracting States : BE, CH, DE, FR, GB, IT, LI, LU, NL, SE**

**1.** An expression control DNA sequence comprising a natural coliphage T5 promoter combined with the E. coli lac operator or a functional part thereof which permits the control of promoter activity.

**2.** An expression control DNA sequence according to claim 1, wherein said coliphage T5 promoter is the $P_{N25}$ promoter.

**3.** An expression control DNA sequence according to claim 1, wherein the lac operator or a functional part thereof overlaps the T5 promoter sequence.

**4.** An expression control DNA sequence according to any one of claims 1 to 3, comprising the functional part of the nucleotide sequence of Fig. 7.

**5.** An expression vector comprising an expression control DNA sequence according to claims 1 to 4.

**6.** An expression vector according to claim 5 which is a plasmid capable of replication in gram-negative and/or gram-positive bacteria.

**7.** An expression vector according to claim 6 which is capable of replication in an E. coli strain.

**8.** An expression vector according to claim 6 which is capable of replication in a B. subtilis strain.

**9.** An expression vector of claim 6 or 7 which is pDS2/$P_{N25}x_{/0}$,$t_o2^*$, having the structure as shown in Figure 8a or b, wherein the symbols used are defined as follows:
represents promoters; represents ribosomal binding sites; represents terminator $t_o$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), $\beta$-lactamase (bla) and lac repressor (lacI) or for parts of the coding region for chloramphenicol acetyltransferase (cat'); represents parts of the coding region for β-galactosidase (lac Z').

**10.** An expression vector of claims 6 or 7 which is pDS3/$P_{N25}x_{/0}$,$t_0 2^{+}$, having the structure as shown in Figure 8b, wherein the symbols used are defined as follows: represents promoters; represents ribosomal binding sites; represents terminator $t_0$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), β-lactamase (bla) and lac repressor (lacI) or for parts of the coding region for chloramphenicol acetyltransferase (cat'); represents parts of the coding region for β-galactosidase (lac Z').

**11.** A transformant carrying an expression vector claimed in any one of claims 5 to 10.

**12.** A transformant according to claim 11 which is an E. coli strain.

**13.** A transformant according to claim 11 which is a B. subtilis strain.

**14.** A process for the manufacture of a pro- or eukaryotic polypeptide which process comprises transforming a host with an expression vector containing the DNA sequence coding for said polypeptide operatively linked to an expression control DNA sequence as claimed in any one of claims 1 to 4, culturing the transformant under appropriate conditions of growth and isolting the desired polypeptide from the culture.

**15.** A process according to claim 14, wherein the vector is a plasmid capable of replication in gram-negative and/or gram-positive bacteria.

**16.** A process according to claim 15, wherein the plasmid is capable of replication in an E. coli strain.

**17.** A process according to claim 15, wherein the plasmid is capable of replication in a B. subtilis strain.

**18.** A process according to claim 15 or 16, wherein the plasmid is pDS2/$P_{N25}x_{/0}$,$t_0 2^{+}$, having the structure as shown in Figure 8a or b, wherein the symbols used are defined as follows: represents promoters; represents ribosomal binding sites; represents terminator $t_0$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), β-lactamase (bla) and lac repressor (lacI) or for parts of the coding region for chloramphenicol acetyltransferase (cat'); represents parts of the coding region for β-galactosidase (lac Z').

**19.** A process according to claim 15 or 16, wherein the plasmid is pDS3/$P_{N25}x_{/0}$,$t_0 2^{+}$, having the structure as shown in Figure 8b, wherein the symbols used are defined as follows: represents promoters; represents ribosomal binding sites; represents terminator $t_0$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), β-lactamase (bla) and lac repressor (lacI) or for parts of the coding region for chloramphenicol acetyltransferase (cat'); represents parts of the coding region for β-galactosidase (lac Z').

**20.** A process according to claims 14 to 19, wherein the polypeptide is chloramphenicol acetyltransferase.

**21.** The use of an expression control DNA sequence as claimed in any one of claims 1 to 4 for expressing a pro-or eukaryotic polypeptide.

**Claims for the following Contracting State : AT**

**1.** A process for the preparation of an expression control DNA sequence comprising a coliphage T5 promoter combined with the E. coli lac operator or a functional part thereof which permits the control of promoter activity, which process comprises combining a coliphage T5 promoter in a manner well known in the art with the E. coli lac operator or a functional part thereof to a functional unit.

**2.** A process according to claim 1, wherein said coliphage T5 promoter is the $P_{N25}$ promoter.

**3.** A process according to claim 1, thereby creating an expression control DNA sequence characterized in that the lac operator or a functional part thereof overlaps the T5 promoter sequence.

**4.** A process according to any one of claims 1 to 3, thereby creating an expression control DNA sequence comprising the functional part of the nucleotide sequence of Fig. 7.

**5.** A process for the preparation of an expression vector comprising a DNA portion corresponding to an expression control DNA sequence obtained according to any one of claims 1-4, which process comprises providing said DNA portion and introducing said DNA portion into a convenient expression vector.

**6.** A process according to claim 5, wherein said convenient expression vector is a plasmid capable of replication in gram-negative and/or gram-positive bacteria.

**7.** A process according to claim 6, wherein said plasmid is capable of replication in an E. coli strain.

**8.** A process according to claim 6, wherein said plasmid is capable of replication in a B. subtilis strain.

**9.** The process of claim 6 or 7, wherein said plasmid is pDS2/$P_{N25}x_{/0}$,$t_o2^+$, having the structure as shown in Figure 8a or b, wherein the symbols used are defined as follows: represents promoters; represents ribosomal binding sites; represents terminator $t_o$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), $\beta$-lactamase (bla) and lac repressor (lacI) or for parts of the coding region for chloramphenicol acetyltransferase (cat'); represents parts of the coding region for $\beta$-galactosidase (lac Z').

**10.** An expression vector of claim 6 or 7 which is pDS3/$P_{N25}x_{/0}$,$t_o2^+$, having the structure as shown in Figure 8b, wherein the symbols used are defined as follows: represents promoters; represents ribosomal binding sites; represents terminator $t_o$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), $\beta$-lactamase (bla) and lac repressor (lacI) or for parts of the coding region for chloramphenicol acetyltransferase (cat'); represents parts of the coding region for $\beta$-galactosidase (lac Z').

**11.** A transformant carrying an expression vector claimed in any one of claims 5 to 10.

**12.** A transformant according to claim 11 which is an E. coli strain.

**13.** A transformant according to claim 11 which is a B. subtilis strain.

**14.** A process for the manufacture of a pro- or eukaryotic polypeptide which process comprises transforming a host with an expression vector containing the DNA sequence coding for said polypeptide operatively linked to an expression control DNA sequence as claimed in any one of claims 1 to 4, culturing the tranformant under appropriate conditions of growth and isolating the desired polypeptide from the culture.

**15.** A process according to claim 14, wherein the vector is a plasmid capable of replication in gram-negative and/or gram-positive bacteria.

**16.** A process according to claim 15, wherein the plasmid is capable of replication in an E. coli strain.

**17.** A process according to claim 15, wherein the plasmid is capable of replication in a B. subtilis strain.

**18.** A process according to claim 15 or 16, wherein the plasmid is pDS2/$P_{N25}x_{/0},t_0 2^+$, having the structure as shown in Figure 8a or b, wherein the symbols used are defined as follows: represents promoters; represents ribosomal binding sites; represents terminator $t_0$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), β-lactamase (bla) and lac repressor (lacI) or for parts of the coding region for chloramphenicol acetyltransferase (cat'); represents parts of the coding region for β-galactosidase (lac Z').

**19.** A process according to claim 15 or 16, wherein the plasmid is pDS3/$P_{N25}x_{/0},t_0 2^+$, having the structure as shown in Figure 8b, wherein the symbols used are defined as follows: represents promoters; represents ribosomal binding sites; represents terminator $t_0$; represents lac operator (O) or parts of it; represents promoter $P_{N25}$ ($P_{N25}$); represents region required for DNA-replication (repl.); represents coding region either for dihydrofolat reductase (dhfr), chloramphenicol acetyltransferase (cat), β-lactamase (bla) and lac repressor (lacI) or for parts of the coding region for chloramphenicol acetyltransferase (cat'); represents parts of the coding region for β-galactosidase (lac Z').

**20.** A process according to claims 14 to 19, wherein the polypeptide is chloramphenicol acetyltransferase.

**Revendications**
**Revendications pour les Etats contractants suivants : BE, CH, DE, FR, GB, IT, LI, LU, NL, SE**

**1.** Séquence d'ADN de commande d'expression comprenant un promoteur de coliphage naturel T5 combiné avec un opérateur lac d'E. coli ou une de ses parties fonctionnelles permettant la commande de l'activité de promoteur.

**2.** Séquence d'ADN de commande d'expression selon la revendication 1, dans laquelle ledit promoteur de coliphage T5 est le promoteur $P_{N25}$.

**3.** Séquence d'ADN de commande d'expression selon la revendication 1, dans laquelle l'opérateur lac ou une de ses parties fonctionnelles se chevauche avec la séquence promoteur T5.

**4.** Séquence d'ADN de commande d'expression selon l'une quelconque des revendications 1 à 3, comprenant la partie fonctionnelle de la séquence de nucléotides de la figure 7.

**5.** Vecteur d'expression comprenant une séquence d'ADN de commande d'expression selon les revendications 1 à 4.

**6.** Vecteur d'expression selon la revendication 5 qui est un plasmide capable de réplication dans des bactéries gram-négatives et/ou gram-positives.

**7.** Vecteur d'expression selon la revendication 6 qui est capable de réplication dans une souche d'E. coli.

**8.** Vecteur d'expression selon la revendication 6 qui est capable de réplication dans une souche de B. subtilis.

**9.** Vecteur d'expression de la revendication 6 ou 7 qui est pDS2/$P_{N25}x_{/0},t_0 2^+$, ayant la structure présentée dans la figure 8a ou b, où les symboles utilisés sont définis comme suit: représente des promoteurs; représente des sites de liaison ribosomiques; représente le terminateur $t_0$ ; représente l'opérateur lac (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la β-lactamase (bla) et du répresseur lac (lacI) ou des parties de la région de codage de la chloramphénicol acétyltransférase (cat'); représente des parties de la région de codage de la β-galactosidase (lac Z').

**10.** Vecteur d'expression des revendications 6 ou 7 qui est le $pDS3/P_{N25}x_{/0},t_{0}2^{+}$, ayant la structure présentée dans la figure 8b, où les symboles utilisés sont définis comme suit: représente des promoteurs; représente des sites de liaison ribosomiques; représente le terminateur $t_0$ ; représente l'opérateur lac (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la $\beta$-lactamase (bla) et du répresseur lac (lacl) ou des parties de la région de codage de la chloramphénicol acétyltransférase (cat'); représente des parties de la région de codage de la $\beta$-galactosidase (lac Z').

**11.** Transformant portant un vecteur d'expression revendiqué dans l'une quelconque des revendications 5 à 10.

**12.** Transformant selon la revendication 11 qui est une souche d'E. coli.

**13.** Transformant selon la revendication 11 qui est une souche de B. subtilis.

**14.** Procédé de préparation d'un polypeptide pro-ou eucaryotique, lequel procédé comprend la transformation d'un hôte avec un vecteur d'expression contenant la séquence d'ADN codant pour ledit polypeptide liée de manière opératoire à une séquence d'ADN de commande d'expression telle que revendiquée dans l'une quelconque des revendications 1 à 4, la culture du transformant dans des conditions de croissance appropriées et l'isolement du polypeptide désiré de la culture.

**15.** Procédé selon la revendication 14, dans lequel le vecteur est un plasmide capable de réplication dans les bactéries gram-négatives et/ou gram-positives.

**16.** Procédé selon la revendication 15, dans lequel le plasmide est capable de réplication dans une souche d'E. coli.

**17.** Procédé selon la revendication 15, dans lequel le plasmide est capable de réplication dans une souche de B. subtilis.

**18.** Procédé selon la revendication 15 ou 16, dans lequel le plasmide est $pDS2/P_{N25}x_{/0},t_{0}2^{+}$, ayant la structure présentée dans la figure 8a ou 8b, où les symboles utilisés sont définis comme suit: représente des promoteurs; représente des sites de liaison ribosomiques; représentele terminateur $t_0$ ; représente l'opérateur lac , (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la $\beta$-lactamase (bla) et du répresseur lac (lacl) ou des parties de la région de codage de la chloramphénicol acétyltransférase (cat'); représente des parties de la région de codage de la $\beta$-galactosidase (lac Z').

**19.** Procédé selon la revendication 15 ou 16, dans lequel le plasmide est $pDS3/P_{N25}x_{/0},t_{0}2^{+}$, ayant la structure présentée dans la figure 8b, où les symboles utilisés sont définis comme suit: représente des promoteurs: représente des sites de liaison ribosomiques; représente le terminateur $t_0$ ; représente l'opérateur lac (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la $\beta$-lactamase (bla) et du répresseur lac (lacl) ou des parties de la région de codage de la chloramphénicol acétyltransférase (cat'); représente des parties de la région de codage de la $\beta$-galactosidase (lac Z').

**20.** Procédé selon les revendications 14 à 19, dans lequel le polypeptide est la chloramphénicol acétyltransférase.

**21.** Application d'une séquence d'ADN de commande d'expression telle que revendiquée dans l'une quelconque des revendications 1 à 4 pour exprimer un polypeptide pro- ou eucaryotique.

**Revendications pour l'Etat contractant suivant : AT**

1. Procédé de préparation d'une séquence d'ADN de commande d'expression comprenant un promoteur de coliphage T5 combiné avec l'opérateur lac d'E. coli ou une partie fonctionnelle de cet opérateur qui permet la commande de l'activité de promoteur, lequel procédé comprend la combinaison d'un promoteur de coliphage T5 de façon bien connue des spécialistes avec l'opérateur lac d'E. coli ou une partie fonctionnelle de cet opérateur pour donner une unité fonctionnelle.

2. Procédé selon la revendication 1, dans lequel ledit promoteur de coliphage T5 est le promoteur $P_{N25}$.

3. Procédé selon la revendication 1, créant ainsi une séquence d'ADN de commande d'expression caractérisée en ce que l'opérateur lac ou une de ses parties fonctionnelles se chevauche avec la séquence de promoteur de T5.

4. Procédé selon l'une quelconque des revendication 1 à 3, créant ainsi une séquence d'ADN de commande d'expression comprenant la partie fonctionnelle de la séquence nucléotidique de la figure 7.

5. Procédé de préparation d'un vecteur d'expression comprenant une partie d'ADN correspondant à une séquence d'ADN de commande d'expression obtenue selon l'une quelconque des revendications 1-4, procédé dans lequel on fournit ladite partie d'ADN et on introduit ladite partie d'ADN dans un vecteur d'expression commode.

6. Procédé selon la revendication 5, dans lequel ledit vecteur d'expression commode est un plasmide capable de réplication dans des bactéries gram-négatives et/ou gram-positives.

7. Procédé selon la revendication 6, dans lequel ledit plasmide est capable de réplication dans une souche d'E. coli.

8. Procédé selon la revendication 6, dans lequel ledit plasmide est capable de réplication dans une souche de B. subtilis.

9. Procédé de la revendication 6 ou 7, dans lequel ledit plasmide est pDS2/$P_{N25}x_{/0}$,$t_0 2^{+}$, ayant la structure présentée dans la figure 8a ou b, où les symboles utilisés sont définis comme suit: représente des promoteurs; représente des sites de liaison ribosomiques; représente le terminateur $t_0$ ; représente l'opérateur lac (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la $\beta$-lactamase (bla) et du répresseur lac (lacI) ou des parties de la région de codage de la chloramphénicol acétyltransférase (cat'); représente des parties de la région de codage de la $\beta$-galactosidase (lac Z').

10. Vecteur d'expression de la revendication 6 ou 7 qui est pDS3/$P_{N25}x_{/0}$,$t_0 2^{+}$ ayant la structure présentée dans la figure 8b, où les symboles utilisés sont définis comme suit: représente des promoteurs; représente des sites de liaison ribosomiques; représente le terminateur $t_0$ ; représente l'opérateur lac (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la $\beta$-lactamase (bla) et du répresseur lac (lacI) ou des parties de la région de codage de la chloramphénicol acétyltransférase (cat'); représente des parties de la région de codage de la $\beta$-galactosidase (lac Z').

11. Transformant portant un vecteur d'expression revendiqué dans l'une quelconque des revendications 5 à 10.

12. Transformant selon la revendication 11 qui est une souche d'E. coli.

13. Transformant selon la revendication 11 qui est une souche de B. subtilis.

**14.** Procédé de préparation d'un polypeptide pro-ou eucaryotique, lequel procédé comprend la transformation d'un hôte avec un vecteur d'expression contenant la séquence d'ADN codant pour ledit polypeptide lié de manière opératoire à une séquence d'ADN de commande d'expression telle que revendiquée dans l'une quelconque des revendication 1 à 4, la culture du transformant dans des conditions de croissance appropriées et l'isolement du polypeptide désiré de la culture.

**15.** Procédé selon la revendication 14, dans lequel le vecteur est un plasmide capable de réplication dans les bactéries gram-négatives ou gram-positives.

**16.** Procédé selon la revendication 15, dans lequel le plasmide est capable de réplication dans une souche d'E. coli.

**17.** Procédé selon la revendication 15, dans lequel le plasmide est capable de réplication dans une souche de B. subtilis.

**18.** Procédé selon la revendication 15 ou 16, dans lequel le plasmide est $pDS2/P_{N25}x_{/0},t_{0}2^{+}$ ayant la structure présentée dans la figure 8a ou B, où les symboles utilisés sont définis comme suit: représente des promoteurs; représente des sites de liaison ribosomiques; représente le terminateur $t_0$ ; représente l'opérateur lac (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la $\beta$-lactamase (bla) et du répresseur lac (lacI) ou des parties de la région de codage de la chloramphénicol acétyltransférase (cat'); représente des parties de la région de codage de la $\beta$-galactosidase (lac Z').

**19.** Procédé selon la revendication 15 ou 16, dans lequel le plasmide est $pDS3/P_{N25}x_{/0},t_{0}2^{+}$, ayant la structure présentée dans la figure 8b, où les symboles utilisés sont définis comme suit: représente des promoteurs; représente des sites de liaison ribosomiques; représente le terminateur $t_0$ ; représente l'opérateur lac (O) ou des parties de cet opérateur; représente le promoteur $P_{N25}$ ($P_{N25}$); représente la région nécessaire pour la réplication de l'ADN (répl.); représente la région de codage de la dihydrofolate réductase (dhfr), de la chloramphénicol acétyltransférase (cat), de la $\beta$-lactamase (bla) et du répresseur lac (lacI) ou des parties de la région de codage de la chloramphénicol acétyltransférase (cat'); représente des parties de la région de codage de la $\beta$-galactosidase (lac Z').

**20.** Procédé selon les revendications 14 à 19, dans lequel le polypeptide est la chloramphénicol acétyltransférase.

**Patentansprüche**

**Patentansprüche für folgende Vertragsstaaten : BE, CH, DE, FR, GB, IT, LI, LU, NL, SE**

**1.** Eine Expressionskontroll-DNA-Sequenz, enthaltend einen natürlichen Coliphagen T5 Promotor, der mit dem E. coli lac Operator oder einem funktionellen Teil davon, welche die Kontrolle der Promotoraktivität gestatten, verknüpft ist.

**2.** Eine Expressionskontroll-DNA-Sequenz gemäss Anspruch 1, worin besagter Coliphage T5 Promotor der $P_{N25}$ Promotor ist.

**3.** Eine Expressionskontroll-DNA-Sequenz gemäss Anspruch 1, worin der lac Operator oder ein funktioneller Teil davon die T5 Promotorsequenz überlappt.

**4.** Eine Expressionskontroll-DNA-Sequenz gemäss einem der Ansprüche 1 bis 3, die den funktionellen Teil der Nukleotidsequenz der Abb. 7 enthält.

**5.** Ein Expressionsvektor, enthaltend eine Expressionskontroll-DNA-Sequenz gemäss den Ansprüchen 1 bis 4.

**6.** Ein Expressionsvektor gemäss Anspruch 5, der ein Plasmid ist, welches in gram-negativen und/oder gram-positiven Bakterien replizieren kann.

**7.** Ein Expressionsvektor gemäss Anspruch 6, der in einem E. coli Stamm replizieren kann.

**8.** Ein Expressionsvektor gemäss Anspruch 6, der in einem B. subtilis Stamm replizieren kann.

**9.** Ein Expressionsvektor gemäss Anspruch 6 oder 7 mit der Bezeichnung pDS2/$P_{N25}x_{/0}$,$t_02^+$, der die in Figur 8a oder b gezeigte Struktur aufweist, worin die verwendeten Symbole wie folgt definiert sind: stellt Promotoren dar; stellt Ribosomen-Bindungsstellen dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), $\beta$-Lactamase (bla) und des lac Repressors (lacI) oder Teile der kodierenden Region der Chloramphenicol-Acetyltransferase (cat') dar; stellt Teile der kodierenden Region der $\beta$-Galactosidase (lac Z') dar.

**10.** Ein Expressionsvektor gemäss Anspruch 6 oder 7 mit der Bezeichnung pDS3/$P_{N25}x_{/0}$,$t_02^+$, der die in Figur 8b gezeigte Struktur aufweist, worin die verwendeten Symbole wie folgt definiert sind: stellt Promotoren dar; stellt Ribosomen-Bindungsstellen dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), $\beta$-Lactamase (bla) und des lac Repressors (lacI) oder Teile der kodierenden Region der Chloramphenicol-Acetyltransferase (cat') dar; stellt Teile der kodierenden Region der $\beta$-Galactosidase (lac Z') dar.

**11.** Eine Transformante, die einen Expressionsvektor gemäss einem der Ansprüche 5 bis 10 trägt.

**12.** Eine Transformante gemäss Anspruch 11, die ein E. coli Stamm ist.

**13.** Eine Transformante gemäss Anspruch 11, die ein B. subtilis Stamm ist.

**14.** Ein Verfahren zur Herstellung eines pro- oder eukaryoten Polypeptids, dadurch gekennzeichnet, dass man einen Wirtsorganismus mit einem Expressionsvektor, welcher die für genanntes Polypeptid kodierende DNA-Sequenz operativ an eine Expressionskontroll-DNA-Sequenz der Ansprüche 1 bis 4 geknüpft enthält, transformiert, die Transformante unter geeigneten Wachstumsbedingungen züchtet und das erhaltene Polypeptid aus der Kultur isoliert.

**15.** Ein Verfahren gemäss Anspruch 14, worin der Vektor ein Plasmid ist, das in gram-negativen und/oder gram-positiven Bakterien replizieren kann.

**16.** Ein Verfahren gemäss Anspruch 15, worin das Plasmid in einem E. coli Stamm replizieren kann.

**17.** Ein Verfahren gemäss Anspruch 15, worin das Plasmid in einem B. subtilis Stamm replizieren kann.

**18.** Ein Verfahren gemäss Anspruch 15 oder 16, worin das Plasmid pDS2/$P_{N25}x_{/0}$,$t_02^+$ ist, das die in Figur 8a oder b gezeigte Struktur aufweist, worin die verwendeten Symbole wie folgt definiert sind: stellt Promotoren dar; stellt Ribosomen-Bindungsstellen dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), $\beta$-Lactamase (bla) und des lac Repressors (lacI) oder Teile der kodierenden Region der Chloramphenicol-Acetyltransferase (cat') dar; stellt Teile der kodierenden Region der $\beta$-Galactosidase (lac Z') dar.

**19.** Ein Verfahren gemäss Anspruch 15 oder 16, worin das Plasmid pDS3/$P_{N25}x_{/0}$,$t_02^+$ ist, das die in Figur 8b gezeigte Struktur aufweist, worin die verwendeten Symbole wie folgt definiert sind: stellt Promotoren dar; stellt Ribosomen-Bindungsstell dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), $\beta$-Lactamase (bla) und des

lac Repressors (lacI) oder Teile der kodierenden Region der Chloramphenicol-Acetyltransferase (cat') dar; stellt Teile der kodierenden Region der β-Galactosidase (lac Z') dar.

**20.** Ein Verfahren gemäss einem der Ansprüche 14 bis 19, worin das Polypeptid Chloramphenicol-Acetyltransferase ist.

**21.** Die Verwendung einer Expressionskontroll-DNA-Sequenz gemäss einem der Ansprüche 1-4 zur Expression von pro- oder eukaryoten Polypeptiden.

**Patentansprüche für folgenden Vertragsstaat : AT**

**1.** Ein Verfahren zur Herstellung einer Expressionskontroll-DNA-Sequenz, enthaltend einen Coliphagen T5 Promotor, der mit dem E. coli lac Operator oder einem funktionellen Teil davon, welche die Kontrolle der Promotoraktivität gestatten, verknüpft ist, dadurch gekennzeichnet, dass man einen Coliphagen T5 Promotor mittels bekannten Techniken mit dem E. coli lac Operator oder einem funktionellen Teil davon zu einer funktionellen Einheit verbindet.

**2.** Ein Verfahren gemäss Anspruch 1, worin besagter Coliphage T5 Promotor der $P_{N25}$ Promoter ist.

**3.** Ein Verfahren gemäss Anspruch 1, wodurch eine ExpressionskontrollDNA-Sequenz gebildet wird, die dadurch gekennzeichnet ist, dass der lac Operator oder ein funktioneller Teil davon die T5 Promotorsequenz überlappt.

**4.** Ein Verfahren gemäss einem der Ansprüche 1-3, wodurch eine Expressionskontroll-DNA-Sequenz, enthaltend den funktionellen Teil der Nukleotidsequenz der Abb. 7, gebildet wird.

**5.** Ein Verfahren zur Herstellung eines Expressionsvektors enthaltend einen DNA-Teil, der einer Expressionskontroll-DNA-Sequenz entspricht, die gemäss einem der Ansprüche 1 bis 4 erhalten wurde, dadurch gekennzeichnet, dass man besagten DNA-Teil zur Verfügung stellt und in einen geeigneten Expressionsvektor einbaut.

**6.** Ein Verfahren gemäss Anspruch 5, worin besagter geeigneter Expressionsvektor ein Plasmid ist, welches in gram-negativen und/oder gram-positiven Bakterien replizieren kann.

**7.** Ein Verfahren gemäss Anspruch 6, worin besagtes Plasmid in einem E. coli Stamm replizieren kann.

**8.** Ein Verfahren gemäss Anspruch 6, worin besagtes Plasmid in einem B. subtilis Stamm replizieren kann.

**9.** Ein Verfahren gemäss Anspruch 6 oder 7, worin das Plasmid pDS2/$P_{N25}x_{/0}$,$t_0 2^*$ ist, das die in Figur 8a oder b gezeigte Struktur aufweist, worin die verwendeten Symbole wie folgt definiert sind: stellt Promotoren dar; stellt Ribosomen-Bindungsstellen dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), β-Lactamase (bla) und des lac Repressors (lacI) oder Teile der kodierenden Region der Chloramphenicol-Acetyltransferase (cat') dar; stellt Teile der kodierenden Region der β-Galactosidase (lac Z') dar.

**10.** Ein Expressionsvektor gemäss Anspruch 6 oder 7 mit der Bezeichnung pDS3/$P_{N25}x_{/0}$,$t_0 2^*$, der die in Figur 8b gezeigte Struktur aufweist, worin die verwendeten Symbole wie folgt definiert sind: stellt Promotoren dar; stellt Ribosomen-Bindungsstellen dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), β-Lactamase (bla) und des lac Repressors (lacI) oder Teile der kodierenden Region der Chloramphenicol-Acetyltransferase (cat') dar; stellt Teile der kodierenden Region der β-Galactosidase (lac Z') dar.

**11.** Eine Transformante, die einen Expressionsvektor gemäss einem der Ansprüche 5 bis 10 trägt.

**12.** Eine Transformante gemäss Anspruch 11, die ein E. coli Stamm ist.

**13.** Eine Transformante gemäss Anspruch 11, die ein B. subtilis Stamm ist.

**14.** Ein Verfahren zur Herstellung eines pro- oder eukaryoten Polypeptids, dadurch gekennzeichnet, dass man einen Wirtsorganismus mit einem Expressionsvektor, welcher die für genanntes Polypeptid kodierende DNA-Sequenz operativ an eine Expressionskontroll-DNA-Sequenz der Ansprüche 1 bis 4 geknüpft enthält, transformiert, die Transformante unter geeigneten Wachstumsbedingungen züchtet und das erhaltene Polypeptid aus der Kultur isoliert.

**15.** Ein Verfahren gemäss Anspruch 14, worin der Vektor ein Plasmid ist, das in gram-negativen und/oder gram-positiven Bakterien replizieren kann.

**16.** Ein Verfahren gemäss Anspruch 15, worin das Plasmid in einem E. coli Stamm replizieren kann.

**17.** Ein Verfahren gemäss Anspruch 15, worin das Plasmid in einem B. subtilis Stamm replizieren kann.

**18.** Ein Verfahren gemäss Anspruch 15 oder 16, worin das Plasmid pDS2/$P_{N25}x_{/0}$,$t_0 2^{+}$ ist, das die in Figur 8a oder b gezeigte Struktur aufweist, worin die verwendeten Symbole wie folgt definiert sind: stellt Promotoren dar; stellt Ribosomen-Bindungsstellen dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), β-Lactamase (bla) und des lac Repressors (lacI) oder Teile der kodierenden Region der Chloramphenicol-Acetyltransferase (cat') dar; stellt Teile der kodierenden Region der β-Galactosidase (lac Z') dar.

**19.** Ein Verfahren gemäss Anspruch 15 oder 16, worin das Plasmid pDS3/$P_{N25}x_{/0}$,$t_0 2^{+}$ ist, das die in Figur 8b gezeigte Struktur aufweist, worin die verwendeten Symbole wie folgt definiert sind: stellt Promotoren dar; stellt Ribosomen-Bindungsstellen dar; stellt den Terminator $t_0$ dar; stellt den lac Operator (O) oder Teile davon dar; stellt den Promotor $P_{N25}$ ($P_{N25}$) dar; stellt die für die DNA-Replikation erforderliche Region (repl.) dar, stellt entweder kodierende Bereiche der Dihydrofolatreduktase (dhfr), Chloramphenicol-Acetyltransferase (cat), β-Lactamase (bla) und des lac Repressors (lacI) oder Teile der kodierenden Region der Chloramphenicol-Acetyltransferase (cat') dar; stellt Teile der kodierenden Region der β-Galactosidase (lac Z') dar.

**20.** Ein Verfahren gemäss den Ansprüchen 14 bis 19, worin das Polypeptid Chloramphenicol-Acetyltransferase ist.

Fig. la

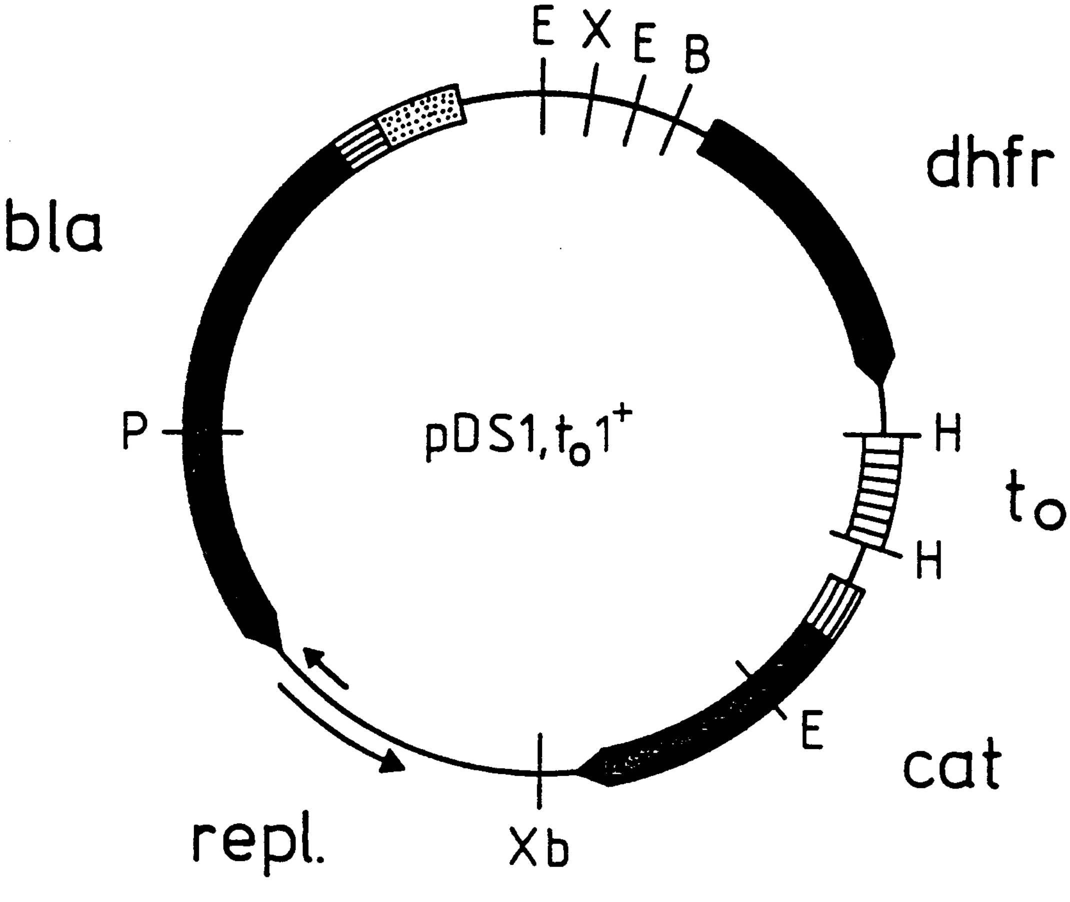

Fig. 1b

| | 10 | 20 | 30 | 40 | 50 |
|---|---|---|---|---|---|
| 0 | GAATTCCTCG | AGGAATTCCG | GATCCGGCAT | CATGGTTCGA | CCATTGAACT |
| 50 | GCATCGTCGC | CGTGTCCCAA | AATATGGGGA | TTGGCAAGAA | CGGAGACCTA |
| 100 | CCCTGGCCTC | CGCTCAGGAA | CGAGTTCAAG | TACTTCCAAA | GAATGACCAC |
| 150 | AACCTCTTCA | GTGGAAGGTA | AACAGAATCT | GGTGATTATG | GGTAGGAAAA |
| 200 | CCTGGTTCTC | CATTCCTGAG | AAGAATCGAC | CTTTAAAGGA | CAGAATTAAT |
| 250 | ATAGTTCTCA | GTAGAGAACT | CAAAGAACCA | CCACGAGGAG | CTCATTTTCT |
| 300 | TGCCAAAAGT | TTGGATGATG | CCTTAAGACT | TATTGAACAA | CCGGAATTGG |
| 350 | CAAGTAAAGT | AGACATGGTT | TGGATAGTCG | GAGGCAGTTC | TGTTTACCAG |
| 400 | GAAGCCATGA | ATCAACCAGG | CCACCTTAGA | CTCTTTGTGA | CAAGGATCAT |
| 450 | GCAGGAATTT | GAAAGTGACA | CGTTTTTCCC | AGAAATTGAT | TTGGGGAAAT |
| 500 | ATAAACTTCT | CCCAGAATAC | CCAGGCGTCC | TCTCTGAGGT | CCAGGAGGAA |
| 550 | AAAGGCATCA | AGTATAAGTT | TGAAGTCTAC | GAGAAGAAAG | ACTAACAGGA |
| 600 | AGATGCTTTC | AAGTTCTCTG | CTCCCCTCCT | AAAGCTATGC | ATTTTTATAA |
| 650 | GACCATGGGA | CTTTTGCTGG | CTTTAGATCC | GGCCAAGCTT | GGACTCCTGT |
| 700 | TGATAGATCC | AGTAATGACC | TCAGAACTCC | ATCTGGATTT | GTTCAGAACG |
| 750 | CTCGGTTGCC | GCCGGGCGTT | TTTTATTGGT | GAGAATCCAA | GCTTGGCGAG |
| 800 | ATTTTCAGGA | GCTAAGGAAG | CTAAAATGGA | GAAAAAAATC | ACTGGATATA |
| 850 | CCACCGTTGA | TATATCCCAA | TGGCATCGTA | AAGAACATTT | TGAGGCATTT |
| 900 | CAGTCAGTTG | CTCAATGTAC | CTATAACCAG | ACCGTTCAGC | TGGATATTAC |
| 950 | GGCCTTTTTA | AAGACCGTAA | AGAAAAATAA | GCACAAGTTT | TATCCGGCCT |
| 1000 | TTATTCACAT | TCTTGCCCGC | CTGATGAATG | CTCATCCGGA | ATTCCGTATG |
| 1050 | GCAATGAAAG | ACGGTGAGCT | GGTGATATGG | GATAGTGTTC | ACCCTTGTTA |
| 1100 | CACCGTTTTC | CATGAGCAAA | CTGAAACGTT | TTCATCGCTC | TGGAGTGAAT |
| 1150 | ACCACGACGA | TTTCCGGCAG | TTTCTACACA | TATATTCGCA | AGATGTGGCG |
| 1200 | TGTTACGGTG | AAAACCTGGC | CTATTTCCCT | AAAGGGTTTA | TTGAGAATAT |
| 1250 | GTTTTTCGTC | TCAGCCAATC | CCTGGGTGAG | TTTCACCAGT | TTTGATTTAA |
| 1300 | ACGTGGCCAA | TATGGACAAC | TTCTTCGCCC | CCGTTTTCAC | CATGGGCAAA |
| 1350 | TATTATACGC | AAGGCGACAA | GGTGCTGATG | CCGCTGGCGA | TTCAGGTTCA |
| 1400 | TCATGCCGTC | TGTGATGGCT | TCCATGTCGG | CAGAATGCTT | AATGAATTAC |
| 1450 | AACAGTACTG | CGATGAGTGG | CAGGGCGGGG | CGTAATTTTT | TTAAGGCAGT |
| 1500 | TATTGGTGCC | CTTAAACCCC | TGGGGTAATG | ACTCTCTAGA | GC |

2068

pBR322 — A

4359

Fig. 2

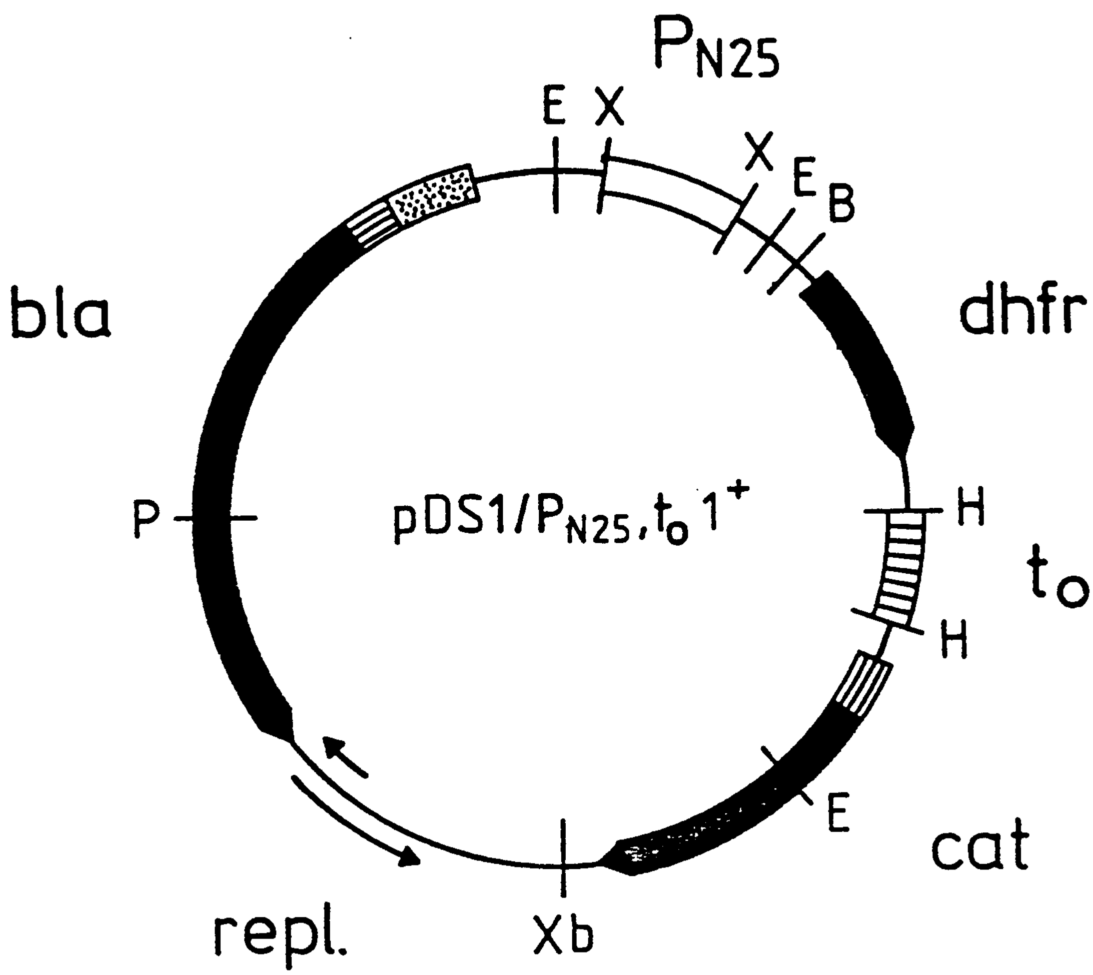


```
              10         20         30         40         50

     XhoI
  0  CTCGAGGCTG GCATCCCTAA CATATCCGAA TGGTTACTTA AACAACGGAG

 50  GACTAGCGTA TCCCTTCGCA TAGGGTTTGA GTTAGATAAA GTATATGCTG

                          HinfI
100  AACTTTCTTC TTTGCTCAAA GAATCATAAA AAATTTATTT GCTTTCAGGA

                        HinfI
150  AAATTTTTCT GTATAATAGA TTCATAAATT TGAGAGAGGA GTTTAAATAT

                                                 XhoI
200  GGCTGGTTCT CGCAGAAAGA AACATATCCA TGAAATCCCG CCTCGAG
```

Fig. 3

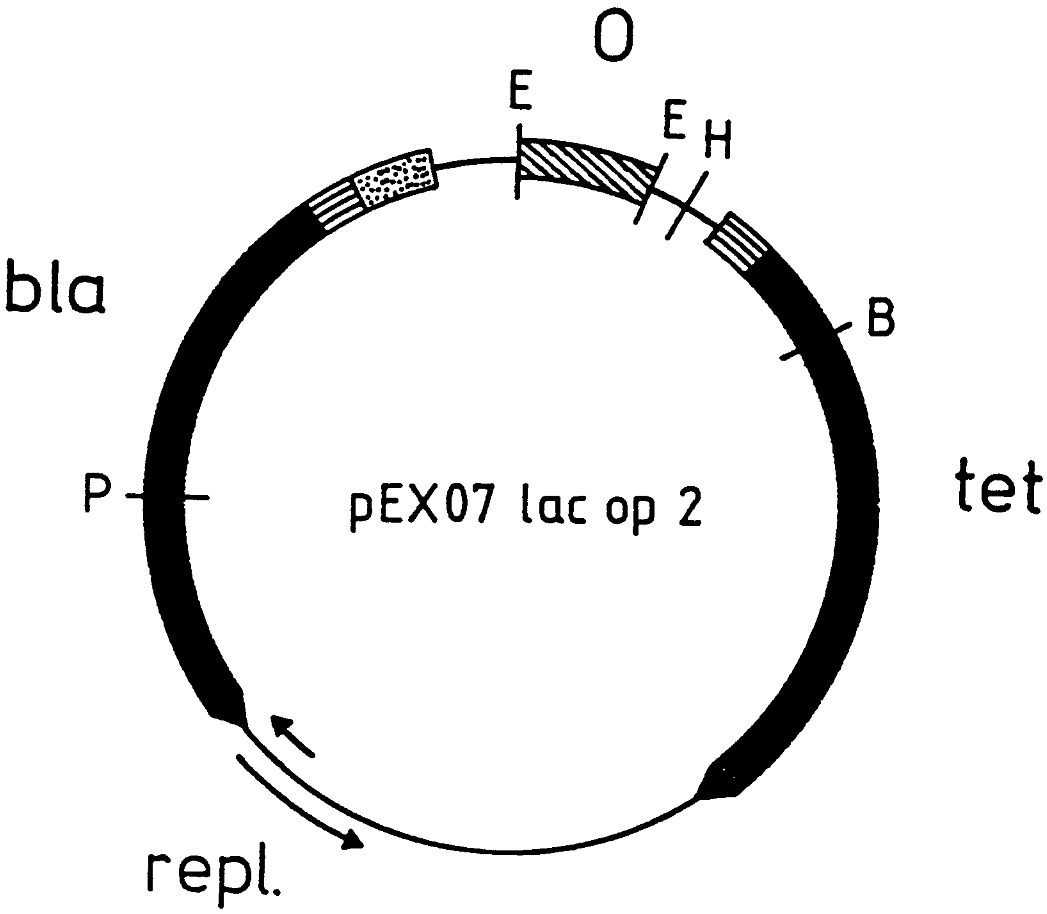


operator

EcoRI EcoRI HindIII

GAATTCAAATTGTGAGCGGATAACAATTTGAATTCCAAGCTT

Fig. 4a

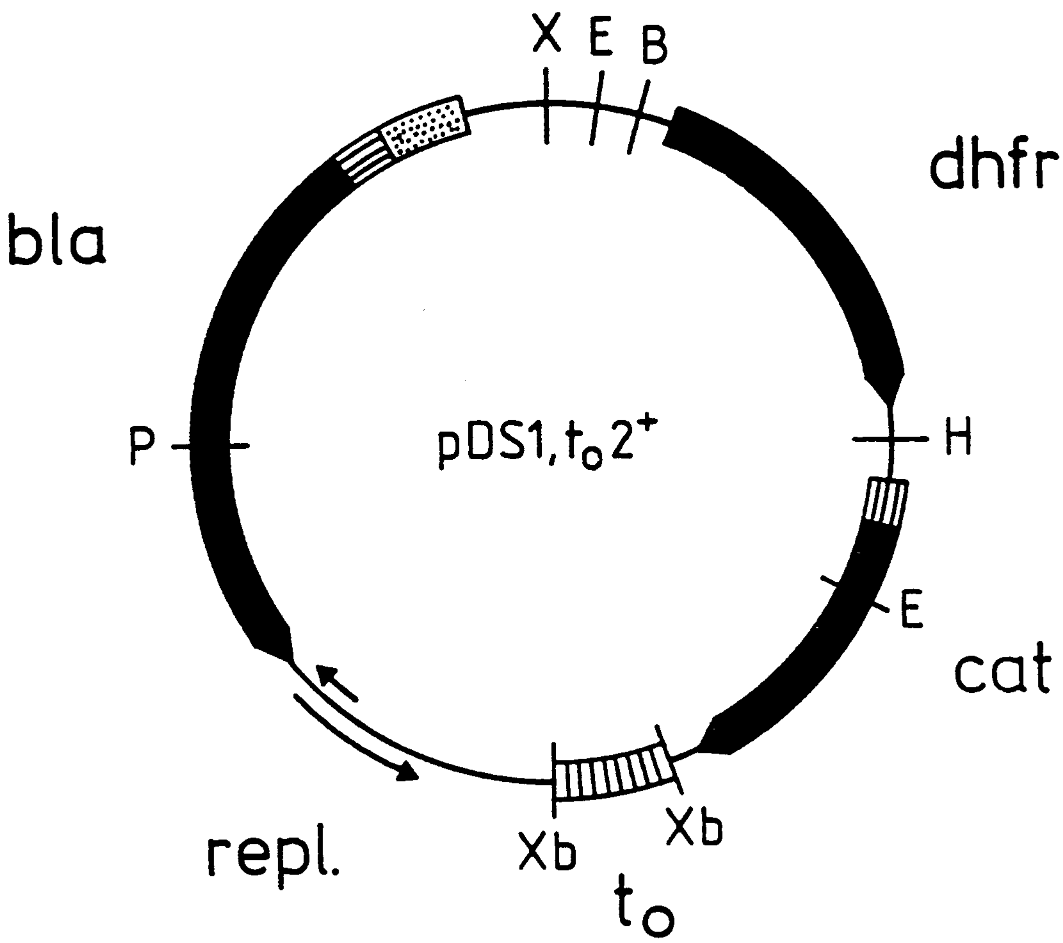

Fig. 4b

```
             10         20         30         40         50
   0 CCTCGAGGAA TTCCGGATCC GGCATCATGG TTCGACCATT GAACTGCATC
  50 GTCGCCGTGT CCCAAAATAT GGGGATTGGC AAGAACGGAG ACCTACCCTG
 100 GCCTCCGCTC AGGAACGAGT TCAAGTACTT CCAAAGAATG ACCACAACCT
 150 CTTCAGTGGA AGGTAAACAG AATCTGGTGA TTATGGGTAG GAAAACCTGG
 200 TTCTCCATTC CTGAGAAGAA TCGACCTTTA AAGGACAGAA TTAATATAGT
 250 TCTCAGTAGA GAACTCAAAG AACCACCACG AGGAGCTCAT TTTCTTGCCA
 300 AAAGTTTGGA TGATGCCTTA AGACTTATTG AACAACCGGA ATTGGCAAGT
 350 AAAGTAGACA TGGTTTGGAT AGTCGGAGGC AGTTCTGTTT ACCAGGAAGC
 400 CATGAATCAA CCAGGCCACC TTAGACTCTT TGTGACAAGG ATCATGCAGG
 450 AATTTGAAAG TGACACGTTT TTCCCAGAAA TTGATTTGGG GAAATATAAA
 500 CTTCTCCCAG AATACCCAGG CGTCCTCTCT GAGGTCCAGG AGGAAAAAGG
 550 CATCAAGTAT AAGTTTGAAG TCTACGAGAA GAAAGACTAA CAGGAAGATG
 600 CTTTCAAGTT CTCTGCTCCC CTCCTAAAGC TATGCATTTT TATAAGACCA
 650 TGGGACTTTT GCTGGCTTTA GATCCGGCCA AGCTTGGCGA GATTTTCAGG
 700 AGCTAAGGAA GCTAAAATGG AGAAAAAAAT CACTGGATAT ACCACCGTTG
 750 ATATATCCCA ATGGCATCGT AAAGAACATT TTGAGGCATT TCAGTCAGTT
 800 GCTCAATGTA CCTATAACCA GACCGTTCAG CTGGATATTA CGGCCTTTTT
 850 AAAGACCGTA AAGAAAAATA AGCACAAGTT TTATCCGGCC TTTATTCACA
 900 TTCTTGCCCG CCTGATGAAT GCTCATCCGG AATTCCGTAT GGCAATGAAA
 950 GACGGTGAGC TGGTGATATG GGATAGTGTT CACCCTTGTT ACACCGTTTT
1000 CCATGAGCAA ACTGAAACGT TTTCATCGCT CTGGAGTGAA TACCACGACG
1050 ATTTCCGGCA GTTTCTACAC ATATATTCGC AAGATGTGGC GTGTTACGGT
1100 GAAAACCTGG CCTATTTCCC TAAAGGGTTT ATTGAGAATA TGTTTTTCGT
1150 CTCAGCCAAT CCCTGGGTGA GTTTCACCAG TTTTGATTTA AACGTGGCCA
1200 ATATGGACAA CTTCTTCGCC CCCGTTTTCA CCATGGGCAA ATATTATACG
1250 CAAGGCGACA AGGTGCTGAT GCCGCTGGCG ATTCAGGTTC ATCATGCCGT
1300 CTGTGATGGC TTCCATGTCG GCAGAATGCT TAATGAATTA CAACAGTACT
1350 GCGATGAGTG GCAGGGCGGG GCGTAATTTT TTTAAGGCAG TTATTGGTGC
1400 CCTTAAACGC CTGGGGTAAT GACTCTCTAG AGACTCCTGT TGATAGATCC
1450 AGTAATGACC TCAGAACTCC ATCTGGATTT GTTCAGAACG CTCGGTTGCC
1500 GCCGGGCGTT TTTTATTGGT GAGAATCTCT AGAGCC
                                        2068
        pBR322                                    A
                                                  4358
```

Fig. 5a

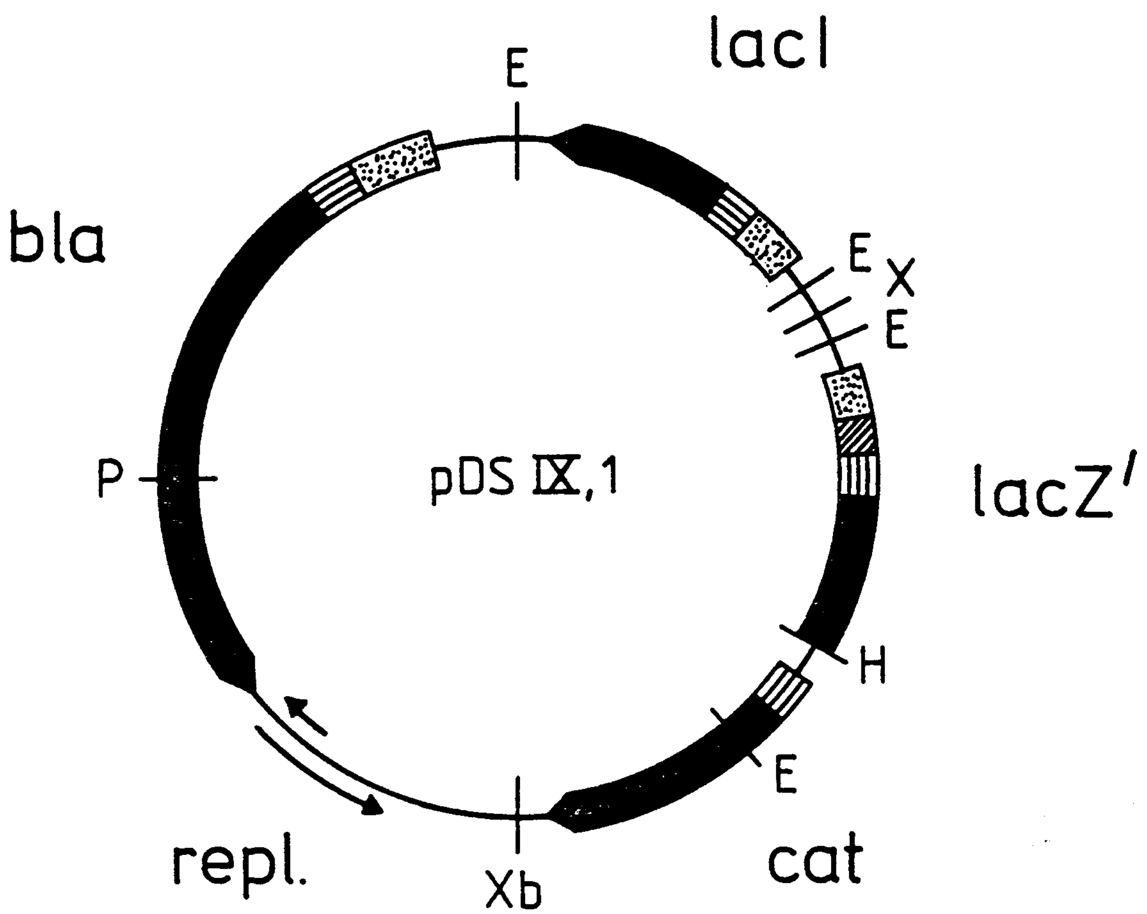

Fig. 5b

| | 10 | 20 | 30 | 40 | 50 |
|---|---|---|---|---|---|
| 0 | GAATTCGCGC | TAACTTACAT | TAATTGCGTT | GCGCTCACTG | CCCGCTTTCC |
| 50 | AGTCGGGAAA | CCTGTCGTGC | CAGCTGCATT | AATGAATCGG | CCAACGCGCG |
| 100 | GGGAGAGGCG | GTTTGCGTAT | TGGGCGCCAG | GGTGGTTTTT | CTTTTCACCA |
| 150 | GTGAGACGGG | CAACAGCTGA | TTGCCCTTCA | CCGCCTGGCC | CTGAGAGAGT |
| 200 | TGCAGCAAGC | GGTCCACGCT | GGTTTGCCCC | AGCAGGCGAA | AATCCTGTTT |
| 250 | GATGGTGGTT | AACGGCGGGA | TATAACATGA | GCTGTCTTCG | GTATCGTCGT |
| 300 | ATCCCACTAC | CGAGATATCC | GCACCAACGC | GCAGCCCGGA | CTCGGTAATG |
| 350 | GCGCGCATTG | CGCCCAGCGC | CATCTGATCG | TTGGCAACCA | GCATCGCAGT |
| 400 | GGGAACGATG | CCCTCATTCA | GCATTTGCAT | GGTTTGTTGA | AAACCGGACA |
| 450 | TGGCACTCCA | GTCGCCTTCC | CGTTCCGCTA | TCGGCTGAAT | TTGATTGCGA |
| 500 | GTGAGATATT | TATGCCAGCC | AGCCAGACGC | AGACGCGCCG | AGACAGAACT |
| 550 | TAATGGGCCC | GCTAACAGCG | CGATTTGCTG | GTGACCCAAT | GCGACCAGAT |
| 600 | GCTCCACGCC | CAGTCGCGTA | CCGTCTTCAT | GGGAGAAAAT | AATACTGTTG |
| 650 | ATGGGTGTCT | GGTCAGAGAC | ATCAAGAAAT | AACGCCGGAA | CATTAGTGCA |
| 700 | GGCAGCTTCC | ACAGCAATGG | CATCCTGGTC | ATCCAGCGGA | TAGTTAATGA |
| 750 | TCAGCCCACT | GACGCGTTGC | GCGAGAAGAT | TGTGCACCGC | CGCTTTACAG |
| 800 | GCTTCGACGC | CGCTTCGTTC | TACCATCGAC | ACCACCACGC | TGGCACCCAG |
| 850 | TTGATCGGCG | CGAGATTTAA | TCGCCGCGAC | AATTTGCGAC | GGCGCGTGCA |
| 900 | GGGCCAGACT | GGAGGTGGCA | ACGCCAATCA | GCAACGACTG | TTTGCCCGCC |
| 950 | AGTTGTTGTG | CCACGCGGTT | GGGAATGTAA | TTCAGCTCCG | CCATCGCCGC |
| 1000 | TTCCACTTTT | TCCCGCGTTT | TCGCAGAAAC | GTGGCTGGCC | TGGTTCACCA |
| 1050 | CGCGGGAAAC | GGTCTGATAA | GAGACACCGG | CATACTCTGC | GACATCGTAT |
| 1100 | AACGTTACTG | GTTTCACATT | CACCACCCTG | AATTGACTCT | CTTCCGGGCG |
| 1150 | CTATCATGCC | ATACCGCGAA | AGGTTTTGCA | CCATTCGATG | GTGTCAACGT |
| 1200 | AAATGCATGC | CGCTTCGCCT | TCGCGCGCGA | ATTCCTCCAG | GAATTCCTCG |
| 1250 | AACCGCCTCC | TGCAACTCTC | TCAGGGCCAG | GCGGTGAAGG | GCAATCAGCT |
| 1300 | GTTGCCCGTC | TCGCTGGTGA | AAAGAAAAAC | CACCCTGGCG | CCCAATACGC |
| 1350 | AAACCGCCTC | TCCCCGCGCG | TTGGCCGATT | CATTAATGCA | GCTGGCACGA |
| 1400 | CACGTTTCCC | GACTGGAAAG | CGGGCAGTGA | GCGCAACGCA | ATTAATGTGA |
| 1450 | GTTAGCTCAC | TCATTAGGCA | CCCCAGGCTT | TACACTTTAT | GCTTCCGGCT |
| 1500 | CGTATGTTGT | GTGGAATTGT | GAGCGGATAA | CAATTTCACA | CAGGAAACAG |
| 1550 | CTATGACCAT | GATTACGGAT | TCACTGGCCG | TCGTTTTACA | ACGTCGTGAC |
| 1600 | TGGGAAAACC | CTGGCGTTAC | CCAACTTAAT | CGCCTTGCAG | CACATCCCCC |
| 1650 | CTTTCGCCAGC | TGGCGTAATA | GCGAAGAGGC | CCGCACCGAT | CGCCCTTCCC |
| 1700 | AACAGTTGCG | CAGCCTGAAT | GGCGAATGGC | GCTTTGCCTG | GTTTCCGTCG |

Fig. 5c

| | 10 | 20 | 30 | 40 | 50 |
|---|---|---|---|---|---|
| 1750 | AAGCTTGGCG | AGATTTTCAG | GAGCTAAGGA | AGCTAAAATG | GAGAAAAAAA |
| 1800 | TCACTGGATA | TACCACCGTT | GATATATCCC | AATGGCATCG | TAAAGAACAT |
| 1850 | TTTGAGGCAT | TTCAGTCAGT | TGCTCAATGT | ACCTATAACC | AGACCGTTCA |
| 1900 | GCTGGATATT | ACGGCCTTTT | TAAAGACCGT | AAAGAAAAAT | AAGCACAAGT |
| 1950 | TTTATCCGGC | CTTTATTCAC | ATTCTTGCCC | GCCTGATGAA | TGCTCATCCG |
| 2000 | GAATTCCGTA | TGGCAATGAA | AGACGGTGAG | CTGGTGATAT | GGGATAGTGT |
| 2050 | TCACCCTTGT | TACACCGTTT | TCCATGAGCA | AACTGAAACG | TTTTCATCGC |
| 2100 | TCTGGAGTGA | ATACCACGAC | GATTTCCGGC | AGTTTCTACA | CATATATTCG |
| 2150 | CAAGATGTGG | CGTGTTACGG | TGAAAACCTG | GCCTATTTCC | CTAAAGGGTT |
| 2200 | TATTGAGAAT | ATGTTTTTCG | TCTCAGCCAA | TCCCTGGGTG | AGTTTCACCA |
| 2250 | GTTTTGATTT | AAACGTGGCC | AATATGGACA | ACTTCTTCGC | CCCCGTTTTC |
| 2300 | ACCATGGGCA | AATATTATAC | GCAAGGCGAC | AAGGTGCTGA | TGCCGCTGGC |
| 2350 | GATTCAGGTT | CATCATGCCG | TCTGTGATGG | CTTCCATGTC | GGCAGAATGC |
| 2400 | TTAATGAATT | ACAACAGTAC | TGCGATGAGT | GGCAGGGCGG | GGCGTAATTT |
| 2450 | TTTTAAGGCA | GTTATTGGTG | CCCTTAAACG | CCTGGGGTAA | TGACTCTCTA |
| 2500 | GAGC | | | | |

2068

pBR322 A

4359

Fig. 6a

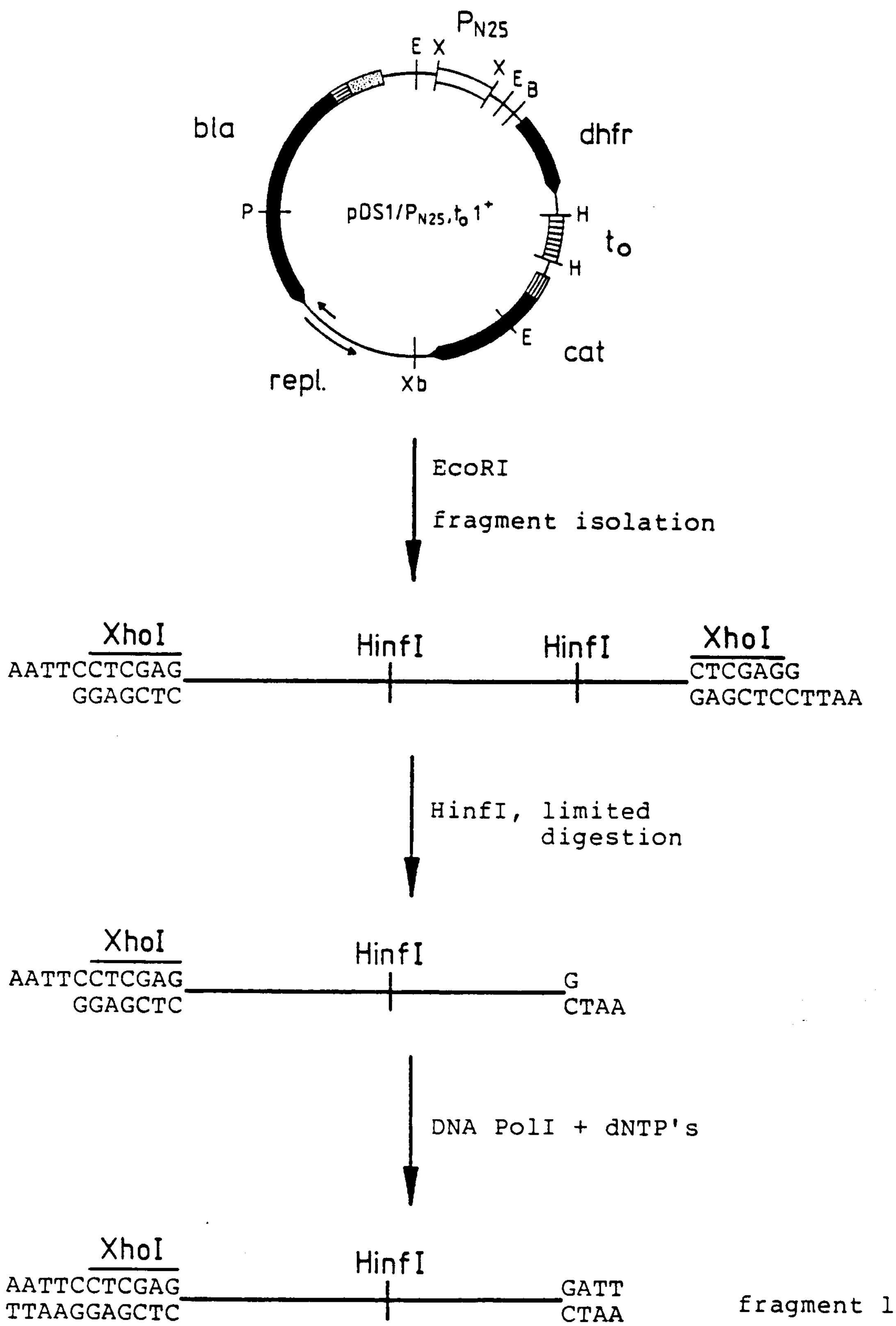
P N25
E
X
X
E
B
bla
dhfr
P
pDS1/P N25.t o 1+
H
t o
H
E
cat
repl.
Xb
EcoRI
fragment isolation
XhoI
HinfI
HinfI
XhoI
AATTCCTCGAG
GGAGCTC
CTCGAGG
GAGCTCCTTAA
HinfI, limited digestion
XhoI
HinfI
AATTCCTCGAG
GGAGCTC
G
CTAA
DNA PolI + dNTP's
XhoI
HinfI
AATTCCTCGAG
TTAAGGAGCTC
GATT
CTAA
fragment 1

Fig. 6b

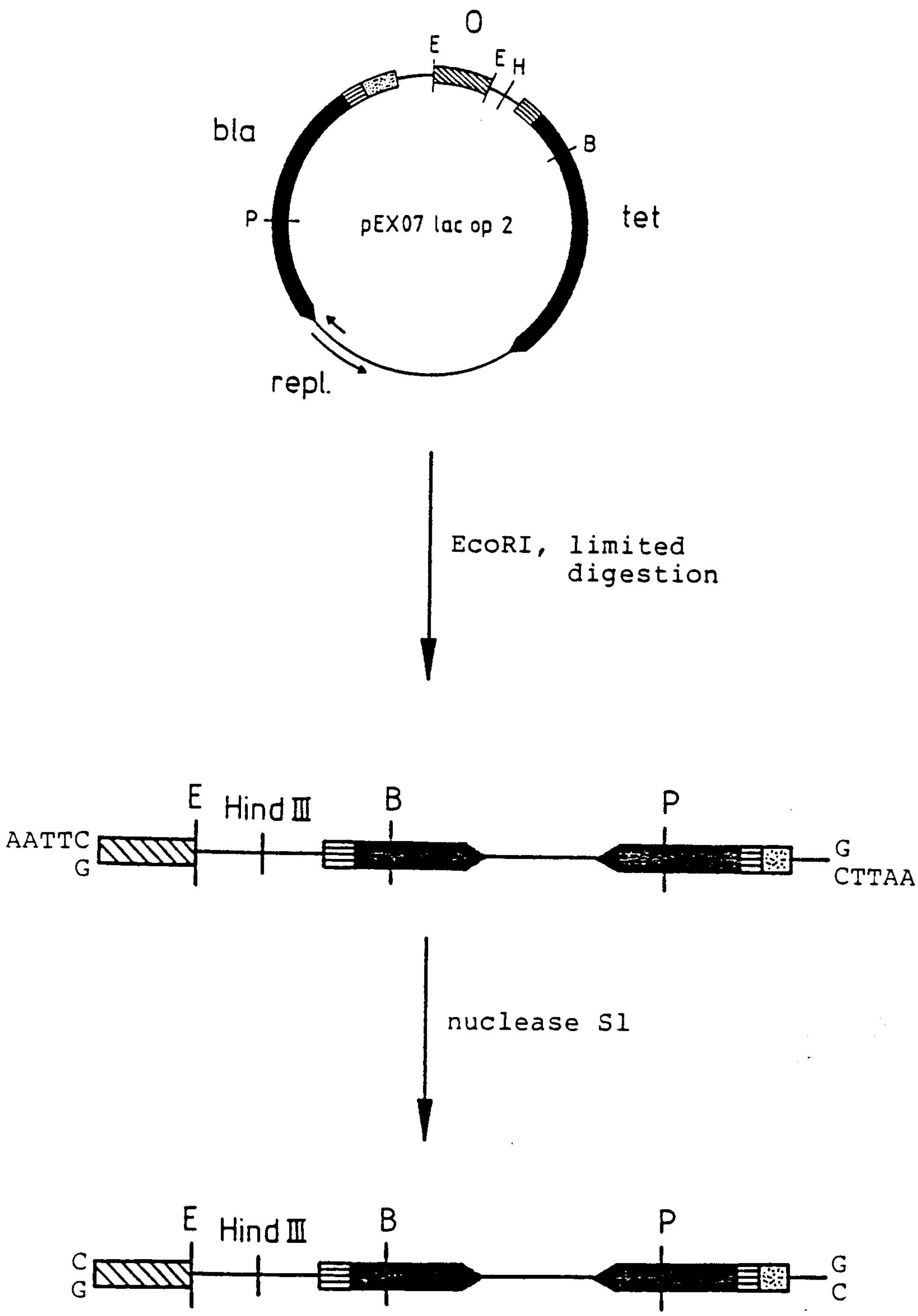


fragment 2

Fig. 6c

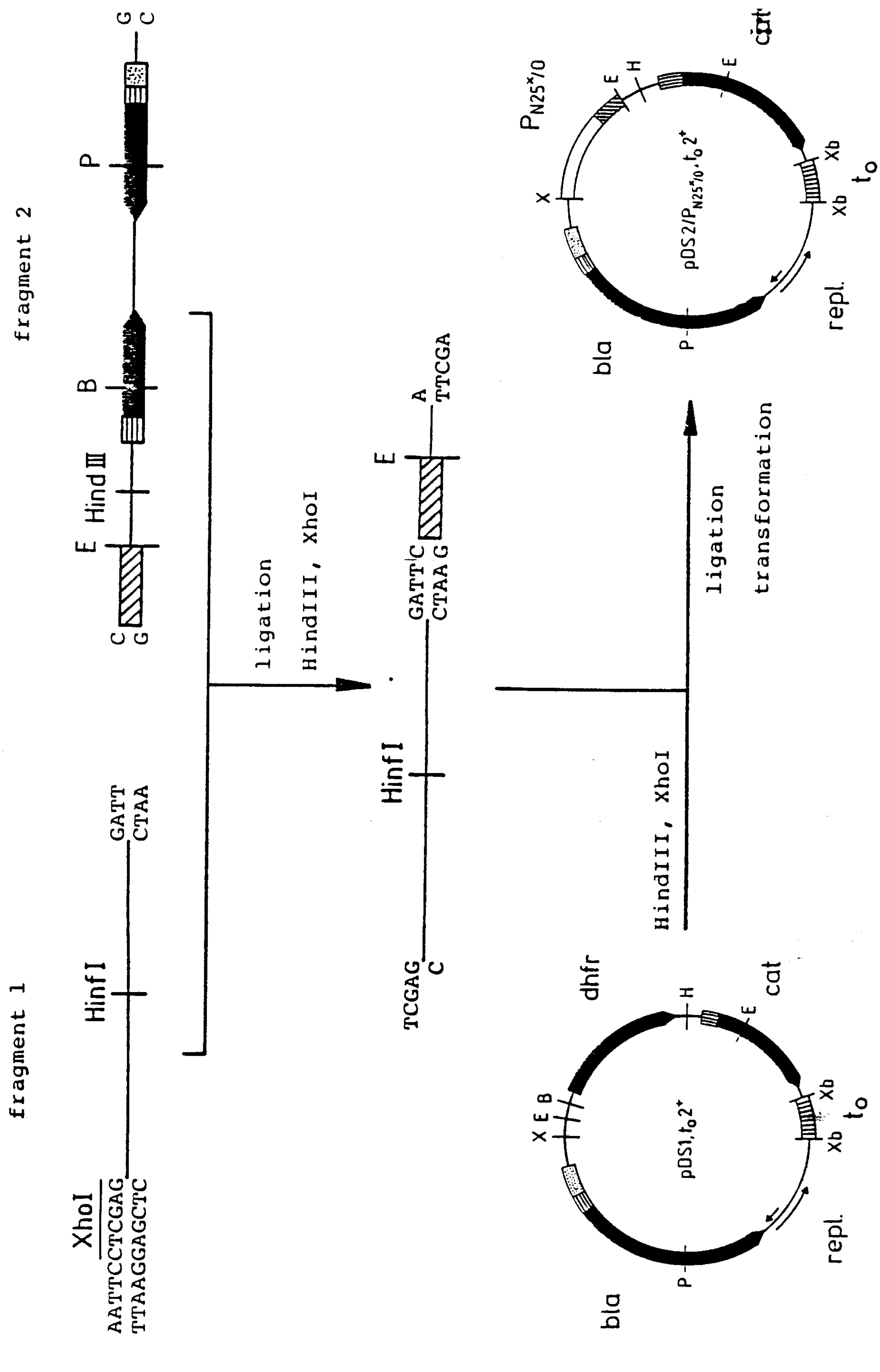
fragment 1
fragment 2
XhoI
AATTCCTCGAG
TTAAGGAGCTC
HinfI
GATT
CTAA
E
HindIII
B
P
ligation
HindIII, XhoI
TCGAG
C
GATTC
CTAAG
A
TTCGA
ligation
transformation
bla
dhfr
cat
repl.
Xb
P
X
H
pDS1,t0 2+
pDS2/PN25x/0,t0 2+
PN25x/0

Fig. 7

| | 10 | 20 | 30 | 40 | 50 |
|---|---|---|---|---|---|
| | XhoI | | | | |
| 0 | CTCGAGGCTG | GCATCCCTAA | CATATCCGAA | TGGTTACTTA | AACAACGGAG |
| 50 | GACTAGCGTA | TCCCTTCGCA | TAGGGTTTGA | GTTAGATAAA | GTATATGCTG |
| | | | HinfI | | |
| 100 | AACTTTCTTC | TTTGCTCAAA | GAATCATAAA | AAATTTATTT | GCTTTCAGGA |
| | | HinfI | | | EcoRI |
| 150 | AAATTTTTCT | GTATAATAGA | TTCAAATTGT | GAGCGGATAA | CAATTTGAATTC |

Fig. 8a

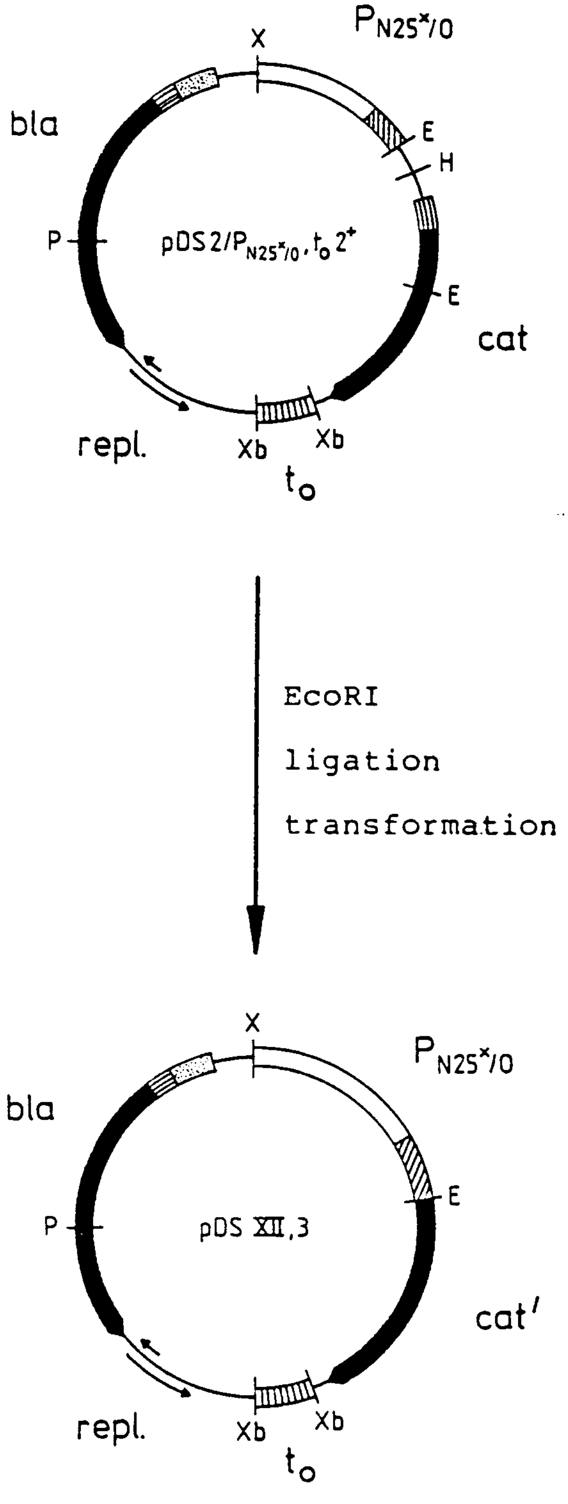
X
PN25*/0
bla
E
H
P
pDS2/PN25*/0.to2+
E
cat
repl.
Xb
Xb
to
EcoRI
ligation
transformation
X
PN25*/0
bla
E
P
pDS XII,3
cat'
repl.
Xb
Xb
to

Fig. 8b

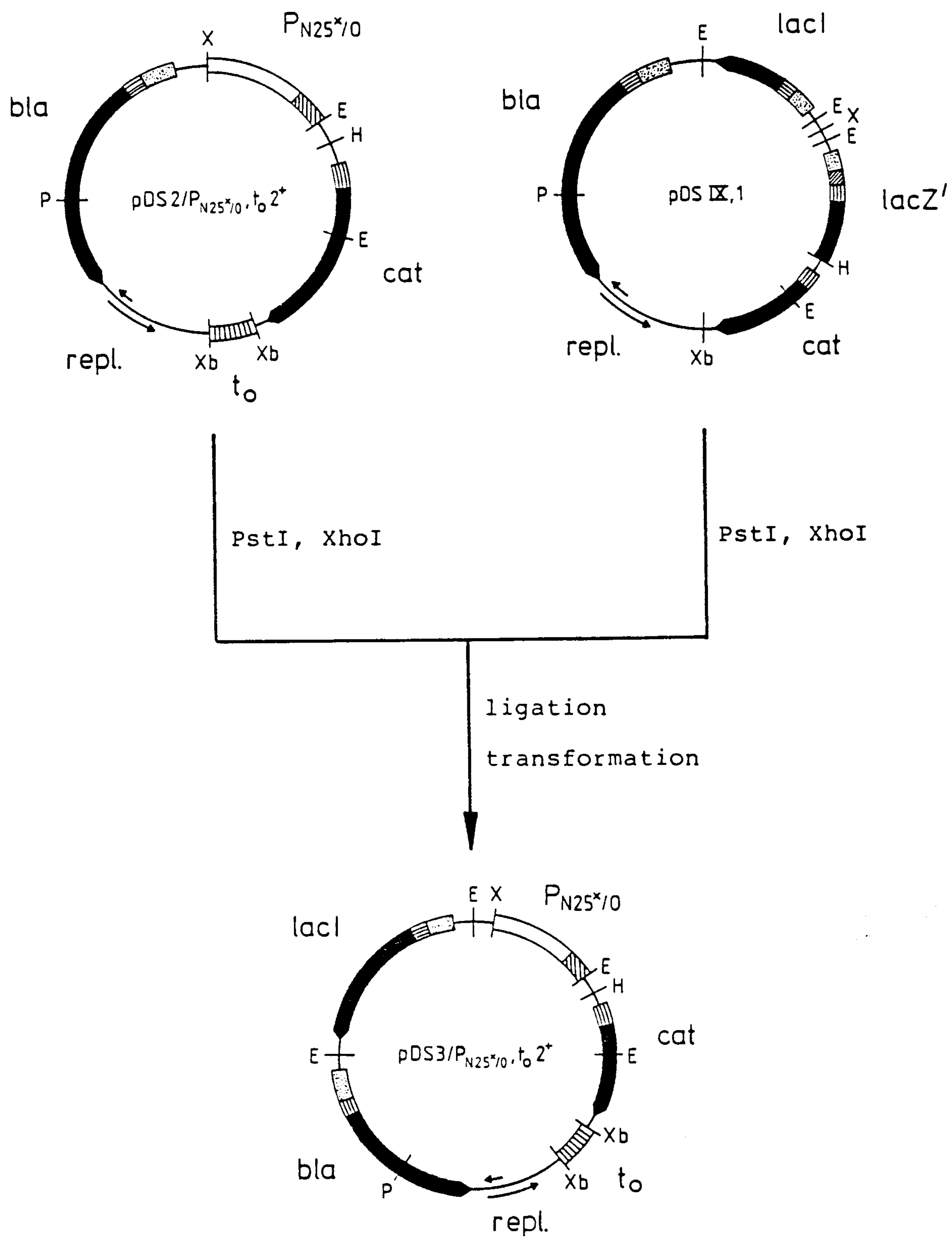
bla
X
PN25*/0
E
H
P
pDS2/PN25*/0,to2+
E
cat
repl.
Xb
Xb
to
E
lacI
bla
E
X
E
P
pDS IX,1
lacZ'
H
E
repl.
Xb
cat
PstI, XhoI
PstI, XhoI
ligation
transformation
E X
PN25*/0
lacI
E
H
cat
E
pDS3/PN25*/0,to2+
E
Xb
bla
P
Xb
to
repl.

Fig. 8c

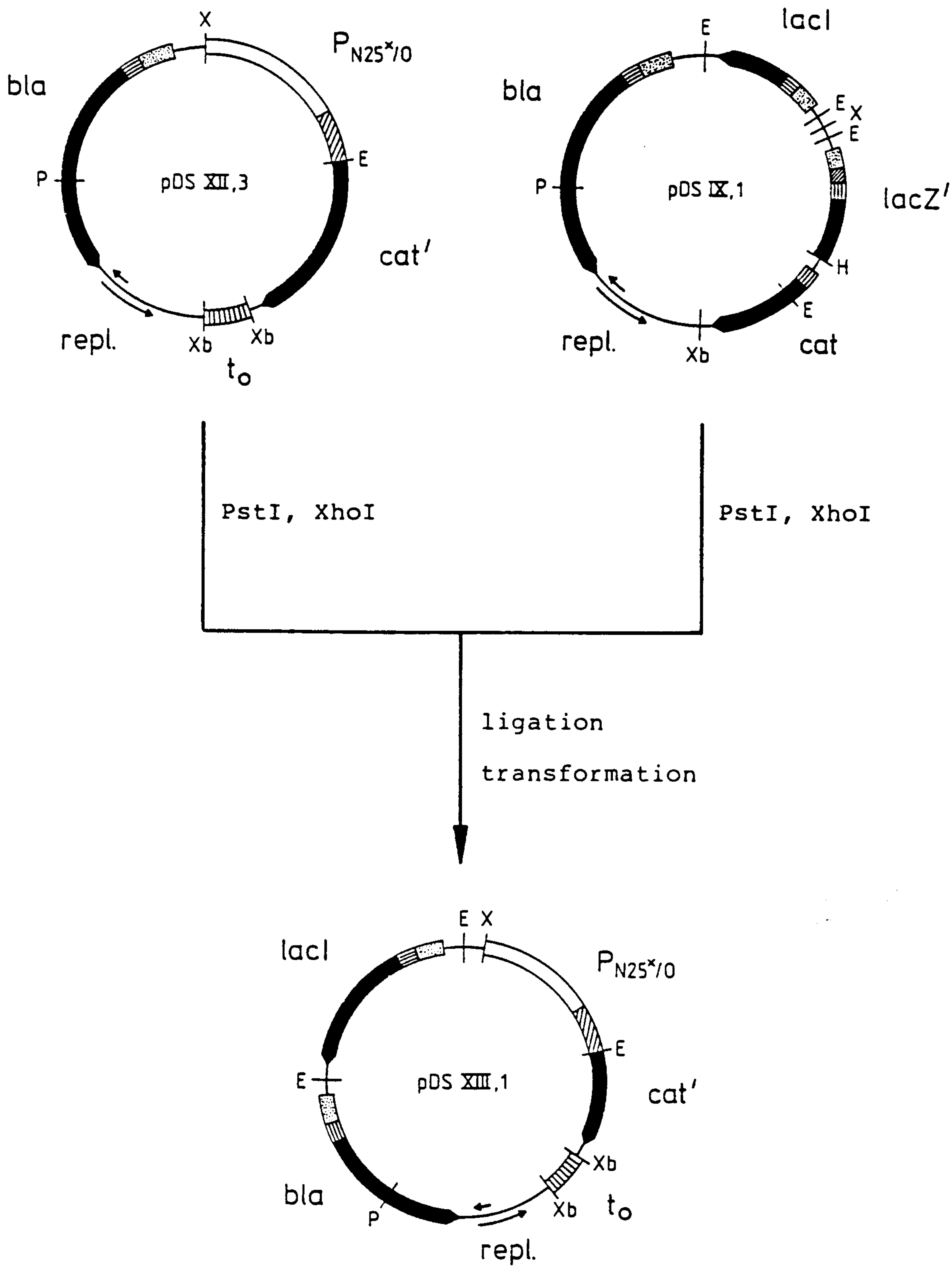
X
$P_{N25^x/0}$
bla
E
P
pDS XII,3
cat'
repl.
Xb
Xb
$t_o$
E
lacI
bla
E
X
E
P
pDS IX,1
lacZ'
H
E
repl.
Xb
cat
PstI, XhoI
PstI, XhoI
ligation
transformation
E X
lacI
$P_{N25^x/0}$
E
E
pDS XIII,1
cat'
Xb
bla
P
Xb
$t_o$
repl.

Fig. 9